(19) **Europäisches Patentamt / European Patent Office / Office européen des brevets**

(11)  **EP 1 789 131 B1**

(12)  # FASCICULE DE BREVET EUROPEEN

(45) Date de publication et mention
de la délivrance du brevet:
**19.10.2016 Bulletin 2016/42**

(21) Numéro de dépôt: **05798449.4**

(22) Date de dépôt: **29.08.2005**

(51) Int Cl.:
***A61N 1/36*** *(2006.01)*

(86) Numéro de dépôt international:
**PCT/FR2005/002161**

(87) Numéro de publication internationale:
**WO 2006/027473 (16.03.2006 Gazette 2006/11)**

(54) **DISPOSITIF DE RÉPARTITION DE COURANT ENTRE DES CATHODES D'UNE ÉLECTRODE MULTIPOLAIRE, NOTAMMENT D'UN IMPLANT**

VORRICHTUNG ZUR VERTEILUNG VON STROM ZWISCHEN KATHODEN EINER MULTIPOLAREN ELEKTRODE, INSBESONDERE EINES IMPLANTATS

DEVICE FOR DISTRIBUTING POWER BETWEEN CATHODES OF A MULTIPOLAR ELECTRODE, IN PARTICULAR OF AN IMPLANT

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **03.09.2004 FR 0409351**

(43) Date de publication de la demande:
**30.05.2007 Bulletin 2007/22**

(73) Titulaires:
• **INRIA - Institut National de Recherche en Informatique et en Automatique
78153 Le Chesnay Cedex (FR)**
• **CENTRE NATIONAL DE
LA RECHERCHE SCIENTIFIQUE -CNRS-
75794 Paris Cedex 16 (FR)**
• **Universite de Montpellier II
34095 Montpellier Cedex 5 (FR)**

(72) Inventeurs:
• **ANDREU, David
F-34070 MONTPELLIER (FR)**

• **BERNARD, Serge
F-34680 Saint Georges d'Orques (FR)**
• **BERTRAND, Yves
F-34090 Montpellier (FR)**
• **CATHERAS, Guy
F-34790 Grabels (FR)**
• **GALY, Jérôme
F-34990 Juvignac (FR)**
• **GUIRAUD, David
F-34000 Montpellier (FR)**
• **TECHER, Jean-Denis
F-94100 Saint Maur des Fossés (FR)**

(74) Mandataire: **Cabinet Netter
36, avenue Hoche
75008 Paris (FR)**

(56) Documents cités:
WO-A-02/089913      WO-A1-93/23114
FR-A- 2 374 024      FR-A- 2 587 624
US-A- 5 954 758

**EP 1 789 131 B1**

**Description**

L'invention concerne le domaine de l'alimentation en courant d'électrodes multipolaires, comportant au moins une anode et au moins deux cathodes, et notamment celles faisant partie d'implants utilisés pour exciter ou stimuler, par exemple, une zone du cerveau, un muscle lisse ou strié, un nerf efférent ou afférent, un organe sensoriel, ou plus généralement un élément du système nerveux d'un humain ou d'un animal.

Dans certains domaines, il est indispensable d'utiliser des électrodes multipolaires pour parvenir à exciter efficacement une ou plusieurs zones, selon un schéma prédéfini. C'est par exemple le cas dans le domaine de la stimulation électrique fonctionnelle, laquelle constitue pour l'instant la principale voie de restauration du mouvement de membres paralysés : cette stimulation est destinée à activer un ou plusieurs muscles par excitation localisée directe et/ou par excitation indirecte d'un nerf au moyen d'un stimulus électrique.

Actuellement, ce type de stimulation s'effectue au moyen d'un implant central placé à l'intérieur du corps. En raison de la grande complexité de la gestion d'un mouvement, différentes électrodes excitatrices doivent être utilisées, si bien que de nombreux fils sont nécessaires au raccordement des électrodes à l'électronique de commande des implants. Ces fils devant fréquemment traverser des articulations, les opérations chirurgicales nécessaires à l'implantation sont particulièrement difficiles, ce qui limite le nombre de zones d'excitation. En outre, la présence de fils fragilise les implants et limite également le nombre d'électrodes qu'ils peuvent commander. Par ailleurs, le contrôle de la forme et des paramètres, notamment temporels, définissant les stimuli électriques, requiert une importance particulière.

Le document WO 02/089913 décrit un système d'implant cochléaire comprenant un microphone, des moyens de traitement du signal convertissant des signaux issus du microphone en une pluralité de signaux de fréquences distinctes, et des électrodes pour distribuer ces signaux aux neurones de la cochlée, le système étant implantable et pouvant recevoir un signal externe.

Le document US 5 954 758 décrit un système de stimulation neuromusculaire

dans lequel une commande d'entrée fournit un signal de commande qui indique un mouvement ou un groupe de mouvements physiologiques. Des moyens déterminent des impulsions à partir de ce signal et génèrent des trains d'impulsions adaptées au signal de commande vers des électrodes pour stimuler les muscles.

Le document WO 93/23114 un système pour un dispositif de fourniture de médicament par ionophorèse qui comprend un circuit de commande de courant comprenant une pluralité de sources de courant chacune couplée à une électrode et choisie pour fournir un courant constant, quelle que soit l'impédance de la peau du patient.

L'invention a donc notamment pour but d'améliorer la situation.

Elle propose notamment à cet effet un dispositif (ou étage de sortie) *selon la revendication 1*dédié à la répartition de courant entre n cathodes d'au moins une électrode multipolaire de stimulation comportant en outre au moins une anode, n étant supérieur ou égal à deux.

Ce dispositif se caractérise par le fait qu'il comporte un miroir de courant reconfigurable comportant n sorties couplées respectivement aux n cathodes et agencé pour délivrer sur ces n sorties n fractions complémentaires d'un courant de commande, de valeurs respectives choisies et sensiblement constantes en présence d'une variation d'amplitude du courant de commande, afin de permettre une localisation spatiale sensiblement constante de la stimulation.

On entend ici par « fractions complémentaires » des fractions dont la somme est égale à la valeur du courant de commande (Idac), comme par exemple Idac/3, Idac/6, Idac/6 et Idac/3.

Le dispositif selon l'invention peut comporter d'autres caractéristiques qui pourront être prises séparément ou en combinaison, et notamment :

- le miroir de courant multi-sorties reconfigurable peut être de type modulaire,

  ➢ il peut alors comporter un convertisseur courant-tension couplé à p convertisseurs tension-courant à transconductance programmable,

    o i) le convertisseur courant-tension peut comprendre au moins une borne d'entrée chargée d'absorber un courant, une borne de masse et une borne de sortie et être agencé pour établir une différence de potentiel choisie entre la borne de sortie et la borne de masse, fonction du courant absorbé,
    o ii) chaque convertisseur tension-courant à transconductance programmable peut comprendre au moins une borne d'entrée, une borne de masse, une borne de sortie chargée d'absorber un courant et un bus de commande chargé de recevoir des signaux logiques,
    ∘ iii) la borne d'entrée du convertisseur courant-tension est alors reliée à une borne d'entrée du miroir de courant multi-sorties reconfigurable modulaire,
    o iv) la borne de masse du convertisseur courant-tension et les bornes de masse des p convertisseurs tension-courant à transconductance programmable sont reliées à la borne de masse M du miroir de courant multi-sorties reconfigurable modulaire,

o v) la borne d'entrée de chacun des p convertisseurs tension-courant à transconductance programmable est reliée à la borne de sortie du convertisseur courant-tension,
o vi) la borne de sortie de chacun des p convertisseurs tension-courant à transconductance programmable est reliée à l'une des sorties du miroir de courant multi-sorties reconfigurable modulaire, et
o vii) le bus de commande de chaque convertisseur tension-courant à transconductance programmable est relié à des sous-bus de commande du miroir de courant multi-sorties reconfigurable modulaire.

➢ le convertisseur courant-tension et les p convertisseurs tension-courant à transconductance programmable présentent préférentiellement des architectures appariées,

- le miroir de courant multi-sorties reconfigurable peut être du type dit « à répartiteur »,

➢ il peut alors comporter un convertisseur courant-tension couplé à un convertisseur tension-courant et à un répartiteur de courant équilibré contrôlable à m sorties,

o i) le convertisseur courant-tension peut alors comprendre au moins une borne d'entrée chargée d'absorber un courant, une borne de masse et une borne de sortie, et être agencé pour établir une différence de potentiel choisie entre la borne de sortie et la borne de masse, fonction du courant absorbé,
o ii) le convertisseur tension-courant peut comprendre au moins une borne d'entrée, une borne de masse et une borne de sortie propre à absorber un courant,
o iii) lé répartiteur de courant équilibré contrôlable peut comprendre au moins une borne d'entrée chargée de délivrer un courant, un bus de sorties absorbant chacune un courant et un bus de commande recevant des signaux logiques,
o iv) la borne d'entrée du convertisseur courant-tension est alors reliée à une borne d'entrée du miroir de courant multi-sorties reconfigurable à répartiteur,
o v) la borne de masse du convertisseur courant-tension et les bornes de masse du convertisseur tension-courant et du répartiteur de courant équilibré contrôlable sont reliées à une borne de masse du miroir de courant multi-sorties reconfigurable à répartiteur,
o vi) la borne d'entrée du répartiteur de courant équilibré contrôlable est reliée à la borne de sortie du convertisseur tension-courant,
o vii) le bus de commande du répartiteur de courant équilibré contrôlable est relié à un bus de commande du miroir de courant multi-sorties reconfigurable à répartiteur, et
o viii) le bus de sorties du répartiteur de courant équilibré contrôlable est relié à un bus de sortie du miroir de courant multi-sorties reconfigurable à répartiteur,

➢ le convertisseur courant-tension et le convertisseur tension-courant présentent préférentiellement des architectures appariées,

- le rapport entre le courant circulant dans l'anode (égal à la somme des courants délivrés sur les sorties du miroir de courant), et le courant de commande peut être configurable ou non configurable,
- un ensemble de n capacités peuvent assurer chacune le couplage de l'une des sorties avec l'une des cathodes,
- un dispositif de surveillance de tension peut être raccordé aux sorties et chargé de mesurer les tensions respectivement présentes au niveau des sorties du miroir de courant de sorte qu'elles permettent un ajustement de la polarisation anodique de l'électrode multipolaire, via un module d'alimentation haute tension,

➢ il peut comprendre un réseau de convertisseurs analogique-numérique ou bien un réseau de n comparateurs de tension chargés chacun de comparer les n tensions au niveau des sorties du miroir de courant par rapport à une tension de référence commune, ou encore un réseau de 2n comparateurs de tension agencés par paire pour comparer les n tensions au niveau des sorties du miroir de courant par rapport à deux tensions de référence communes,

- un dispositif de contrôle de décharge peut être couplé aux sorties du miroir de courant et à l'anode et être chargé d'établir en fin de stimulation un chemin de conduction entre chacune des sorties du miroir de courant et l'anode, afin d'induire la circulation de n courants de décharge des cathodes vers l'anode,

➢ ces n courants de décharge peuvent provenir des n énergies respectivement accumulées par les n capacités de l'ensemble,

➢ il peut être chargé de limiter chaque courant de décharge à une fraction dé la valeur maximale du courant de stimulation qui est délivré sur la sortie associée.

Un tel dispositif de répartition de courant (ou étage de sortie) peut faire partie avantageusement d'une électronique de commande d'au moins une électrode multipolaire, comprenant au moins une anode et au moins deux cathodes. Cette électronique de commande comprend alors en outre i) un convertisseur numérique-analogique chargé de convertir une consigne d'amplitude de courant en un courant analogique de commande et couplé au dispositif de répartition de courant pour lui fournir le courant de commande, et ii) un module d'alimentation haute tension couplé au moins à l'anode et chargé de polariser cette dernière sous une tension choisie de sorte qu'elle permette la circulation des courants imposés à chaque cathode par le dispositif de répartition de courant.

Le convertisseur numérique-analogique peut présenter une architecture dite « à source de courant unitaire » permettant de garantir la monotonicité de sa fonction de conversion.

Le module d'alimentation haute tension peut être un convertisseur de type « continu-continu ». Dans ce cas, il peut être agencé sous la forme d'un hacheur à stockage inductif (par exemple de type "boost") ou bien comporter une pompe de charges à stockage capacitif, comme par exemple une pompe de Dickson, éventuellement couplée à un multiplexeur. Dans ce dernier cas, le module d'alimentation haute tension peut fonctionner soit en régime continu soit en régime discontinu.

L'invention porte également sur une unité de stimulation répartie (USR), comme par exemple un implant, comportant au moins une électrode multipolaire, comprenant au moins une anode et au moins deux cathodes, et au moins une électronique de commande du type de celle présentée ci-avant.

Cette unité de stimulation répartie peut comprendre un contrôleur numérique (CN) chargé de délivrer la consigne d'amplitude de courant et de définir les valeurs des fractions de courant délivrées sur les sorties du miroir de courant multi-sorties reconfigurable. Dans ce cas, le contrôleur numérique et l'électronique de commande (EC) peuvent par exemple constituer respectivement une partie numérique et une partie analogique d'un ASIC de type mixte.

En outre, le contrôleur numérique peut être chargé de déduire, à partir des valeurs des courants de stimulation imposés, de la tension de sortie du module d'alimentation haute tension et des mesures de tension effectuées par le dispositif de surveillance de tension aux bornes des sorties du miroir de courant multi-sorties reconfigurable, l'impédance de chaque électrode, afin de contrôler la polarisation de l'anode.

L'invention porte également sur une installation de stimulation comprenant au moins une unité de stimulation répartie du type de celle présentée ci-avant, et un contrôleur (CR) chargé d'échanger des données avec chaque unité de stimulation répartie.

Par ailleurs, chaque unité de stimulation répartie et le contrôleur de l'installation peuvent comporter des moyens de transmission par voie d'ondes (ou par bus filaire) et des moyens de gestion chargés de gérer la transmission de données selon un protocole choisi entre ledit contrôleur et chaque unité de stimulation répartie.

contrôleur d'une installation du type de celle présentée ci-avant et au moins une unité de stimulation répartie du type de celle présentée ci-avant, peuvent communiquer via un medium suivant un protocole. Ce protocole se caractérise par le fait qu'il consiste à gérer l'accès au médium selon un principe de droit de parole de groupe(s) d'unités de stimulation répartie à intervalles glissants, basé sur un positionnement automatique d'intervalles temporels dépendant de niveaux de priorité associés respectivement à chaque noeud au sein de son groupe et de caractéristiques topologiques, comme par exemple le débit de données et le temps de propagation.

Cette gestion d'accès au medium est par exemple destinée à optimiser l'exploitation de la bande passante.

Le dispositif de répartition de courant (ou étage de sortie), l'électronique de commande, l'unité de stimulation répartie (implant), l'installation de stimulation et le protocole de communication présentés ci-avant sont particulièrement bien adaptés à la stimulation de nerf(s) et/ou de muscle(s) d'animal ou d'humain.

D'autres caractéristiques et avantages de l'invention apparaîtront à l'examen de la description détaillée ci-après, et des dessins annexés, sur lesquels :

- la figure 1 illustre de façon très schématique un exemple d'installation de stimulation selon l'invention,
- la figure 2 illustre de façon schématique un exemple d'électronique de commande selon l'invention, couplée à une électrode multipolaire et à un contrôleur numérique,
- la figure 3 illustre de façon schématique un premier exemple de module d'alimentation haute tension à hacheur de type « boost », pour une électronique de commande selon l'invention,
- la figure 4 illustre de façon schématique un deuxième exemple de module d'alimentation haute tension à pompe de Dickson, pour une électronique de commande selon l'invention,
- la figure 5 illustre de façon schématique un troisième exemple de module d'alimentation haute tension à pompe de Dickson et multiplexeur, pour une électronique de commande selon l'invention,
- la figure 6 illustre de façon schématique un exemple d'étage de sortie, pour une électronique de commande selon l'invention,
- la figure 7 illustre de façon schématique un exemple de module de contrôle de décharge pour un étage de sortie d'une électronique de commande selon l'invention,

- la figure 8 illustre des exemples de chronogrammes définissant les évolutions temporelles du courant I traversant l'électrode multipolaire et de signaux de contrôle (SA, SB et SC) du module de contrôle de décharge, provenant du contrôleur numérique,
- la figure 9 illustre de façon schématique un exemple d'électrode multipolaire avant enroulement autour d'un nerf (partie de gauche), et une vue en coupe transversale d'un exemple de nerf et de ses fibres nerveuses équipé de ladite électrode multipolaire (partie de droite), dans le cas d'une répartion de courant de type (¼, 0, 0, ¾),
- les figures 10A et 10B illustrent de façon schématique deux exemples de réalisation d'un convertisseur courant-tension pour un miroir de courant multi-sorties reconfigurable d'un étage de sortie d'une électronique de commande selon l'invention,
- les figures 11A à 11C illustrent de façon schématique trois exemples de réalisation d'un convertisseur tension-courant pour un miroir de courant multi-sorties reconfigurable d'un étage de sortie d'une électronique de commande selon l'invention,
- les figures 12A à 12C illustrent de façon schématique trois exemples de réalisation d'un convertisseur tension-courant contrôlable pour un miroir de courant multi-sorties reconfigurable d'un étage de sortie d'une électronique de commande selon l'invention,
- la figure 13 illustre de façon schématique un exemple de réalisation d'un convertisseur tension-courant à transconductance programmable pour un miroir de courant multi-sorties reconfigurable d'un étage de sortie d'une électronique de commande selon l'invention,
- la figure 14 illustre de façon schématique un exemple de réalisation d'un répartiteur de courant équilibré pour un miroir de courant multi-sorties reconfigurable d'un étage de sortie d'une électronique de commande selon l'invention,
- les figures 15A et 15B illustrent de façon schématique deux exemples de réalisation d'un répartiteur de courant équilibré contrôlable, respectivement à TECs à canal N et à TECs à canal N et à canal P, pour un miroir de courant multi-sorties reconfigurable d'un étage de sortie d'une électronique de commande selon l'invention,
- la figure 16 illustre de façon schématique un exemple de réalisation d'un miroir de courant multi-sorties reconfigurable de type modulaire, pour un étage de sortie d'une électronique de commande selon l'invention,
- la figure 17 illustre de façon schématique un exemple de réalisation d'un miroir de courant multi-sorties reconfigurable de type « à répartiteur », pour un étage de sortie d'une électronique de commande selon l'invention,
- la figure 18 illustre de façon plus détaillée un autre exemple de réalisation d'un miroir de courant multi-sorties reconfigurable de type « à répartiteur », pour un étage de sortie d'une électronique de commande selon l'invention,
- la figure 19 illustre de façon schématique les notions de droit de parole individuel et de droit de parole de groupe au sein d'une installation de stimulation selon l'invention,
- la figure 20 illustre de façon schématique le décalage induit par un positionnement relatif,
- la figure 21 illustre de façon schématique le recalage dans le cas d'un positionnement relatif,
- la figure 22 illustre de façon schématique une fenêtre temporelle associée à un groupe d'USRs et sa décomposition en intervalles temporels associés à chacun des USRs dudit groupe,
- la figure 23 illustre de façon schématique le mécanisme de glissement d'intervalle temporel au sein d'un groupe d'USRs,
- la figure 24 illustre de façon schématique un premier exemple de chronogramme d'accès au médium, et
- la figure 25 illustre de façon schématique un second exemple de chronogramme d'accès au médium.

Les dessins annexés pourront non seulement servir à compléter l'invention, mais aussi contribuer à sa définition, le cas échéant.

L'invention est destinée à permettre l'alimentation répartie des cathodes d'au moins une électrode multipolaire.

Dans ce qui suit, on considère, à titre d'exemple illustratif et non limitatif, que l'électrode fait partie d'un implant destiné à être implanté dans un corps humain ou animal afin de stimuler une ou plusieurs zones, comme par exemple un ou plusieurs nerfs et/ou un ou plusieurs muscles. Mais, une telle électrode pourrait être utilisée dans d'autres domaines comme par exemple la stimulation du cerveau, ou la stimulation des systèmes sensoriels au niveau des voies nerveuses afférentes ou des capteurs biologiques eux mêmes.

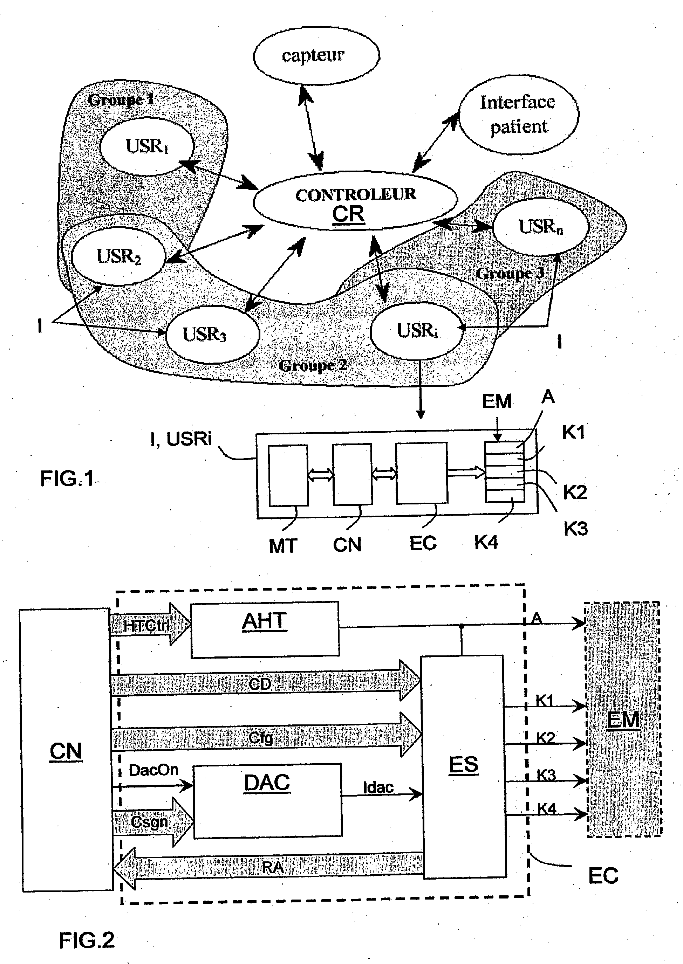

Comme cela est schématiquement illustré sur la figure 1, un implant I (ou USR, Unité de Stimulation Répartie) est essentiellement constitué d'un contrôleur numérique CN couplé, d'une part, à des moyens de transmission MT, et d'autre part, à une électronique de commande EC, elle même couplée à une électrode multipolaire EM comportant au moins une anode A et au moins deux cathodes Ki (i = 1 à 2). Lorsque l'électrode multipolaire EM est dédiée à la stimulation d'un nerf, il est avantageux qu'elle soit agencée sous la forme d'un cylindre et comporte au moins une anode A et par exemple quatre cathodes K1 à K4 (i = 1 à 4). Mais, d'autres formes d'électrodes peuvent être envisagées.

Comme illustré sur la figure 1, l'implant I peut, en compagnie d'un ou plusieurs autres implants, faire partie d'une installation de stimulation IS. Dans ce cas, chaque implant I constitue ce que l'on appelle une unité de stimulation répartie (USR) et l'installation comporte préférentiellement un contrôleur externe (CR) couplé à chaque implant I, de préférence par voie d'ondes (par exemple de type radiofréquence RF), mais un couplage par bus filaire est également envisageable. Ce couplage est destiné. à permettre l'échange de données entre chaque implant I et le contrôleur CR de l'installation IS. Ainsi, le contrôleur CR peut transmettre à chaque implant I des données, définissant par exemple des messages de configuration ou d'interrogation, et éventuellement de l'énergie. Par ailleurs, un implant I peut transmettre au contrôleur CR des données définissant, par exemple, des notifications d'erreur(s) de stimulation ou d'ordre(s) incohérent(s) ou des acquittements.

Une telle transmission peut par exemple se faire en mode paquet asynchrone. Par ailleurs, la transmission, au moins du contrôleur CR vers les implants I, peut se faire soit en mode de diffusion point à multipoint (ou multicast), lorsqu'elle concerne un groupe d'implants de l'installation, soit en mode de diffusion générale (ou broadcast), lorsqu'elle concerne tous les implants de l'installation, soit encore en mode point à point (ou unicast), lorsqu'elle ne concerne qu'un seul implant de l'installation.

L'installation de stimulation peut également comprendre un ou plusieurs capteurs couplés au contrôleur CR et/ou une interface patient également couplée au contrôleur CR et permettant au patient, objet de la stimulation, d'interrompre un programme de stimulation, par exemple.

Par ailleurs, comme on le verra plus loin, les implants I (ou USRs) peuvent être regroupés dynamiquement en différents groupes permettant par exemple de commander simultanément un groupe minimal d'implants I (ou USR) nécessaires à l'obtention d'un mouvement donné, et certains implants (ou USRs) pouvant appartenir à des groupes différents (comme c'est ici le cas des USR2 et USRi).

Par exemple, le lever d'un pied nécessite la contraction simultanée des trois muscles fléchisseurs de la hanche, du genou et de la cheville ; les trois USRs stimulant ces muscles pourront donc être regroupées pour ce mouvement.

On se réfère maintenant à la figure 2 pour présenter les principaux constituants de l'électronique de commande EC d'un implant I selon l'invention. Il est important de noter que l'électronique de commande EC illustrée sur la figure 2 est dédiée à une électrode multipolaire EM comportant quatre cathodes Ki (i = 1 à 4). Mais, l'invention n'est pas limitée à ce type d'électrode multipolaire. Elle concerne en effet tout dispositif de stimulation possédant au moins une anode et au moins deux cathodes.

Afin de faciliter la compréhension de la description, plusieurs définitions sont données ci-dessous :

- un ASIC est ici un circuit intégré spécifique à une application,
- un ASIC mixte est ici un ASIC comprenant des parties de traitement numérique et des parties de traitement analogique,
- Vdd désigne la borne positive de l'alimentation électrique d'un implant I et, indifféremment, la valeur de la tension d'alimentation. Cette dernière est par exemple égale à 3 volts (V) mais toute autre tension, par exemple mieux adaptée au mode de réalisation de l'ASIC, peut être utilisée,
- Gnd désigne la masse électrique, et indifféremment (du fait qu'ils sont connectés) la borne négative de l'alimentation électrique d'un implant I ou la masse électrique ou le substrat de l'ASIC si la technologie de ce dernier impose de relier son substrat au potentiel le plus bas comme c'est le cas en technologie CMOS sur substrat de type P.

Par ailleurs, à l'intérieur d'un bus, tous les signaux portent le nom du bus et sont distingués par un indice placé entre crochets après le nom du bus. En outre, lorsque la taille du bus doit être spécifiée, le nom du bus est suivi d'une paire de crochets contenant l'indice le plus bas et l'indice le plus haut des fils composant le bus, séparés par le caractère ":". La même notation peut être utilisée pour faire référence à une partie d'un bus. Enfin, pour des bus particulièrement complexes, on peut utiliser une notation multidimensionnelle. Quelques exemples de notation sont donnés ci-dessous :

- X désigne aussi bien un signal qu'un bus,
- X[1:10] désigne un bus de 10 signaux,
- X[3] désigne le troisième signal du bus nommé X,
- X[3:5] désigne un bus formé par l'extraction de trois signaux du bus X,
- Y[1:5][1:4] désigne un bus de 20 (5x4) signaux, organisé en 5 sous-bus de 4 signaux chacun,
- Y[3][2] représente un signal, et
- Y[3] est un bus de quatre signaux que l'on peut également écrire Y[3][1:4].

L'électronique de commande EC peut par exemple être réalisée sous la forme d'une partie analogique d'un ASIC mixte dont la partie numérique peut comprendre un contrôleur numérique CN, du type de celui représenté sur la figure 2, lequel ne sera décrit ci-après que par les commandes qu'il est susceptible d'envoyer à l'électronique de commande EC. Les moyens de transmission MT représentés sur la figure 1 ne seront pas non plus décrits ici.

L'électronique de commande EC comprend au moins trois parties.

Une première partie est constituée d'un convertisseur numérique-analogique DAC, chargé de convertir une consigne d'amplitude de courant (Csgn) reçue du contrôleur numérique CN en un courant analogique de sortie Idac.

Une deuxième partie est constituée d'un module d'alimentation haute tension AHT chargé de polariser l'anode A de l'électrode multipolaire EM sous une tension suffisamment élevée pour permettre la circulation du courant imposé à chaque cathode par l'étage de sortie ES (également appelé dispositif de répartition de courant). Cette haute tension peut, par exemple, être engendrée à partir de l'alimentation basse tension Vdd.

Une troisième partie est constituée de l'étage de sortie ES (également appelé dispositif de répartition de courant) qui est destiné principalement à répartir le courant analogique de sortie Idac en fractions et à les transmettre aux différentes cathodes Ki de l'électrode multipolaire EM. Comme on le verra plus loin, cet étage de sortie ES assure également, de préférence, la compensation des charges qui sont accumulées par le courant traversant l'électrode multipolaire EM afin d'assurer que la valeur moyenne du courant de stimulation traversant chaque cathode Ki est nulle.

Les trois parties de l'électronique de commande EC reçoivent l'alimentation électrique Vdd et la masse électrique Gnd.

Le convertisseur DAC est par exemple un convertisseur 8 bits numérique/analogique fonctionnant en mode courant. Le courant peut par exemple être programmé de 0 à 1,25 mA, éventuellement par pas de 5 $\mu$A. Ce convertisseur DAC utilise préférentiellement une architecture dite « à source de courant unitaire » qui garantit la monotonicité de sa fonction de conversion.

Par ailleurs, le convertisseur DAC possède une entrée DacOn qui le contraint, lorsqu'elle est placée au niveau logique « 0 », à délivrer un courant nul (Idac = 0), et qui l'autorise, lorsqu'elle est placée au niveau logique « 1 », à valider un courant de sortie Idac égal à $(2^N - 1) * I\_LSB$. Ici, N représente le nombre de bits du convertisseur (par exemple N = 8 bits) et I_LSB représente le pas de quantification du convertisseur (par exemple 5 pA).

Afin de minimiser la consommation globale, on peut éventuellement utiliser un convertisseur DAC qui minimise, voire même annule, sa consommation lorsque son entrée DacOn est placée au niveau logique « 0 ».

Le module d'alimentation haute tension AHT fournit la tension de polarisation anodique de l'électrode multipolaire EM. Pour la plupart des stimulations cette tension est plus élevée que la tension d'alimentation Vdd. Par conséquent, le module d'alimentation haute tension AHT est préférentiellement un convertisseur de type « continu-continu ». Afin de limiter la puissance consommée, la tension de polarisation anodique peut être fixée « a priori » par le contrôleur numérique CN à partir de l'amplitude du courant de stimulation à appliquer. De plus, l'étage de sortie ES renvoie au contrôleur numérique CN une information RA relative à l'amplitude de la tension aux bornes des générateurs de courant qui commandent les différentes cathodes Ki. Cette information RA peut alors être utilisée pendant la phase de stimulation pour ajuster la polarisation anodique.

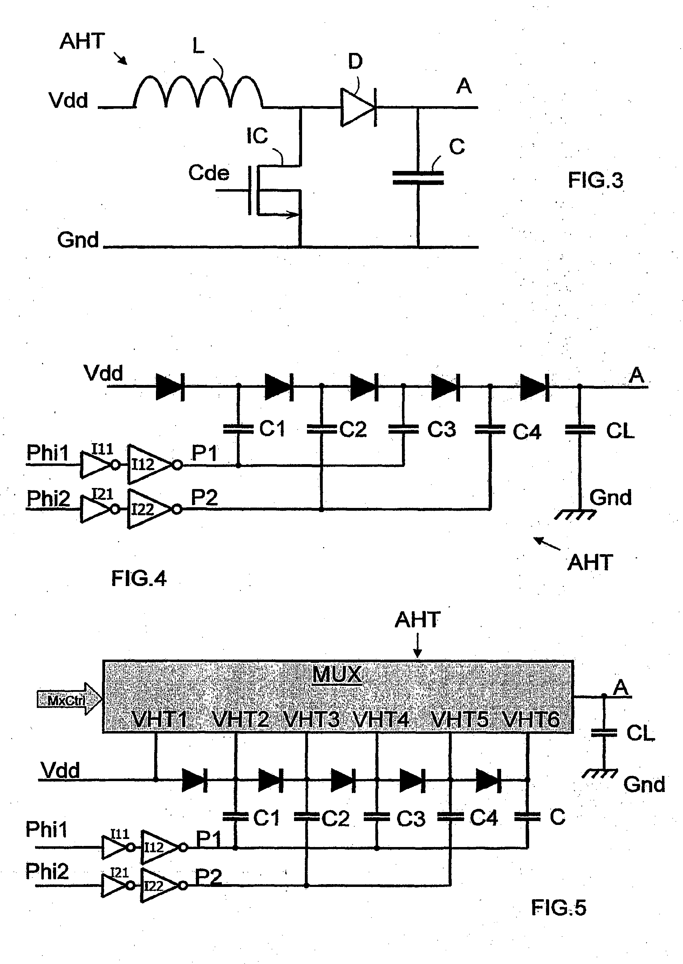

Ce module d'alimentation haute tension AHT peut par exemple être réalisé sous la forme d'un hacheur à stockage inductif (par exemple de type « boost »). Comme le sait l'homme de l'art, un tel hacheur est contrôlé par un unique signal de commande dont la fréquence et le rapport cyclique déterminent la tension de sortie pour une charge donnée. Sur la figure 3 est illustré un exemple de hacheur de type boost utilisant quatre composants : une inductance L, une capacité C, une diode (par exemple de type « schottky » à faible tension de seuil) D et un interrupteur commandé IC. Dans cet exemple dé réalisation, le signal d'entrée Cde constitue à lui seul le faisceau référencé HTCtrl sur la figure 2.

Les composants L, D et C sont préférentiellement des composants discrets externes à l'ASIC. Par ailleurs, l'interrupteur commandé IC peut être soit un composant discret, éventuellement spécialisé, soit un dispositif intégré à l'ASIC, comme par exemple un transistor à effet de champ à grille isolée et canal N, comme illustré sur la figure 3.

Dans une variante de réalisation le module d'alimentation haute tension AHT peut comporter une pompe de charges à stockage capacitif, comme par exemple une pompe de Dickson en technologie CMOS. Un exemple de pompe de Dickson à cinq étages est illustré sur la figure 4. Ici, les diodes sont, en général, réalisées avec des transistors MOS, par exemple à canal N, dont la grille est reliée au drain. On peut aussi utiliser des schémas plus complexes qui permettent de s'affranchir de la tension de seuil. Sur cette figure 4, les références I11, I12, I21 et I22 désignent des inverseurs CMOS alimentés entre Vdd et Gnd et convenablement dimensionnés de manière à pouvoir charger les capacités dans un temps compatible avec la fréquence de fonctionnement envisagée. Lorsque les signaux P1 et P2 sont deux signaux rectangulaires de même fréquence, la tension de sortie à vide de la pompe ne dépend que du nombre d'étages et de la valeur de Vdd. Par ailleurs, la fréquence de fonctionnement commande la résistance de sortie du convertisseur, offrant ainsi un moyen de contrôle de la tension de sortie en charge. Le nombre d'étages est par conséquent imposé par la tension maximale à fournir au point A et le faisceau HTCtrl de la figure 2 est uniquement constitué des signaux d'entrée Phi1 et Phi2.

Dans une autre variante de réalisation, illustrée sur la figure 5, le module d'alimentation haute tension AHT comporte une pompe de Dickson associée à un multiplexeur analogique MUX. En effet, dans une pompe de Dickson, les tensions disponibles sur chaque étage diffèrent de Vdd, si bien que le multiplexeur MUX permet de disposer de toute une gamme de tensions permettant d'ajuster la tension de polarisation anodique. Dans cet exemple de réalisation, le multiplexeur MUX autorise la circulation d'un courant depuis l'une de ses entrées VHTi (ici i = 1 à 6), choisie par le contrôleur numérique CN, vers la sortie A, la circulation d'un courant de A vers l'une des entrées VHTi étant interdite. De cette façon, le contrôleur numérique CN peut adapter la tension anodique, par pas de Vdd, sans avoir à jouer sur la fréquence de fonctionnement. Le faisceau MxCtrl comprend autant de fils que le multiplexeur MUX possède d'entrées VHTi. Lorsque l'on utilise un tel module d'alimentation haute tension AHT, le faisceau HTCtrl de la figure 2 est composé du faisceau MxCtrl associé aux signaux Phi1 et Phi2.

Ce multiplexeur MUX peut, par exemple, être réalisé comme une étoile, dont la sortie A est le centre et dont les entrées VHTi sont les extrémités des branches. Chaque branche de l'étoile peut alors contenir un redresseur commandé (par exemple une diode en série avec un transistor à effet de champ (TEC) à grille isolée). La grille desdits transistors est alors commandée par les signaux logique du faisceau MxCtrl via des adaptateurs de niveau logique, éventuellement alimentés par un étage supplémentaire de la pompe de charge, non représenté ici.

Cette autre variante de réalisation du module d'alimentation haute tension AHT peut être utilisée soit en régime continu, soit en régime discontinu. En régime continu, la pompe est actionnée pendant la stimulation (signaux rectangulaires en opposition de phase sur Phi1 et Phi2). En régime discontinu, la pompe est préchargée avant la stimulation et arrêtée pendant celle-ci. Cela suppose bien entendu que les capacités sont suffisamment importantes (quelques microfarads) pour pouvoir fournir la charge correspondant à une stimulation. Par conséquent, ces capacités peuvent être externes à l'ASIC. Dans le régime discontinu, l'état dans lequel se trouvent les signaux Phi1 et Phi2 conditionne les valeurs de tension VHTi disponibles en entrée du multiplexeur MUX. Le tableau ci-dessous donne un exemple de valeurs de tension VHTi en fonction des états respectifs des signaux Phi1 et Phi2 :

| Phi1/Phi2 | VHT1 | VHT2 | VHT3 | VHT4 | VHT5 | VHT6 |
|---|---|---|---|---|---|---|
| 0/1 | Vdd | Vdd | 3 Vdd | 3 Vdd | 5 Vdd | 5 Vdd |
| 1/0 | Vdd | 2 Vdd | 2 Vdd | 4 Vdd | 4 Vdd | 6 Vdd |

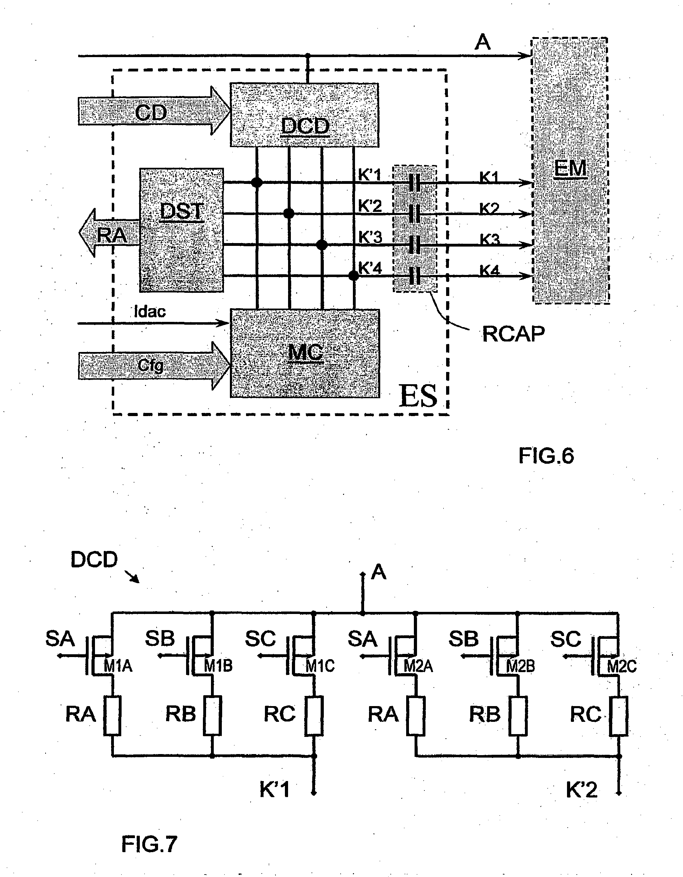

On se réfère maintenant aux figures 6 à 18 pour décrire un exemple de réalisation de l'étage de sortie ES, également appelé dispositif de répartition de courant.

Comme cela est illustré sur la figure 6, l'étage de sortie ES peut, par exemple, être composé de quatre parties : un miroir de courant multi-sorties reconfigurable MC, un dispositif de contrôle de décharge DCD, un dispositif de surveillance dé tension DST et un réseau de capacités RCAP.

Dans ce qui suit, sauf indication contraire, l'expression « miroir de courant » désigne le miroir de courant multi-sorties reconfigurable MC. Par ailleurs, et comme indiqué précédemment, le nombre (n = 4) de cathodes Ki illustré sur la figure 6 n'est qu'un exemple non limitatif, l'invention s'appliquant à tout dispositif d'excitation possédant au moins une anode et au moins deux cathodes.

Le dispositif de surveillance de tension DST est raccordé aux sorties K'i du miroir de courant MC afin de mesurer les tensions respectivement présentes au niveau de ses bornes. Ces mesures sont envoyées, via le faisceau de signaux RA, au contrôleur numérique CN. Ce dernier peut utiliser cette information pour ajuster la polarisation anodique de l'électrode multipolaire EM, via le module d'alimentation haute tension AHT décrit précédemment, afin de minimiser la puissance dissipée dans le miroir de courant MC, tout en lui permettant de fonctionner à la polarisation de sortie optimale. De plus, le contrôleur numérique CN peut déduire de ces mesures l'impédance Zi de chaque électrode Ki, du fait qu'il connaît le courant de stimulation imposé et la tension de sortie du module d'alimentation haute tension AHT.

Ce dispositif de surveillance de tension DST peut être agencé sous la forme d'un réseau de convertisseurs analogique-numérique, mais il peut aussi être, beaucoup plus simplement, agencé sous la forme d'un réseau de n comparateurs de tension (n étant le nombre de cathodes de l'électrode multipolaire EM) comparant les n tensions de sortie du miroir de courant MC par rapport à une tension de référence commune, engendrée de manière interne ou bien imposée de l'extérieur. Dans le cas d'un réseau de n comparateurs, le faisceau RA est tout simplement constitué des n signaux logiques de sortie desdits comparateurs.

L'alimentation anodique commandée de façon numérique est associée à une détection de seuil de tension aux bornes des sources de courant qui commandent les cathodes Ki, de manière à mesurer indirectement la tension aux bornes de l'électrode multipolaire EM et en déduire son impédance, connaissant les valeurs des courants imposés par le miroir de courant MAC. Cela permet de s'affranchir de l'utilisation d'un convertisseur analogique/numérique par cathode Ki combinée à une mesure différentielle, au moyen d'un pôle haute tension du côté de l'anode A, ce qui consommerait beaucoup d'énergie et requerrait une place importante sur un circuit intégré. En outre, cela permet d'évaluer les impédances vues au niveau de chaque cathode Ki. Par exemple, l'identification d'un modèle d'électrode du premier ordre nécessite un compteur de temps et seulement trois mesures.

L'une des contraintes principales que doit respecter un dispositif de stimulation est la nullité de la moyenne du courant de stimulation dans chaque cathode, sous peine de provoquer des lésions au niveau du site de stimulation. Par conséquent, le réseau de capacités RCAP est préférentiellement constitué de n capacités (n étant le nombre de cathodes de l'électrode multipolaire EM) placées en série avec chacune des cathodes Ki de l'électrode multipolaire EM.

A la fin d'une stimulation, les capacités constituant le réseau de capacités RCAP ont accumulé une charge représentant l'intégrale du courant de stimulation ayant circulé dans la cathode rattachée. Le dispositif de contrôle de décharge DCD a donc pour rôle, lorsqu'il est prévu, d'établir un chemin de conduction entre chacun des points K'i et l'anode A de l'électrode multipolaire EM. Il s'ensuit une inversion des rôles de cathode Ki et d'anode A de l'électrode multipolaire EM et la circulation de courants allant des cathodes Ki (jouant donc ici un rôle d'anode) vers l'anode A (jouant donc ici un rôle de cathode) sous l'effet de l'énergie accumulée dans les capacités du réseau RCAP. Lorsque les capacités sont déchargées, il a circulé dans chaque cathode Ki, au cours de la phase de décharge, un courant dont l'intégrale est exactement opposée à l'intégrale du courant de stimulation, sur un cycle stimulation-décharge. La valeur moyenne du courant de stimulation est donc nulle.

Il est préférable que le courant de décharge ne puisse pas être interprété comme une stimulation. En effet, les tissus nerveux présentent une phase de récupération, qui suit immédiatement une stimulation, et au cours de laquelle ils sont insensibles aux stimulations dont l'amplitude reste plus faible que la stimulation initiale, le dispositif de contrôle de décharge DCD est donc préférentiellement agencé de manière à permettre une limitation de chaque courant de décharge à une fraction, par exemple égale à 10%, de l'amplitude maximale du courant de stimulation délivré sur la sortie K'I correspondante (ou associée).

La figure 7 illustre un exemple de réalisation d'un tel dispositif de contrôle de décharge DCD dans le cas d'une électrode multipolaire EM à deux cathodes K1, K2.

Dans cet exemple, les signaux SA, SB et SC constituent le faisceau CD des figures 2 et 6. Ils sont fournis par le contrôleur numérique CN, éventuellement à travers des adaptateurs de niveau logique, alimentés par l'anode A, lesquels permettent de fournir des tensions capables d'assurer un blocage convenable des transistors.

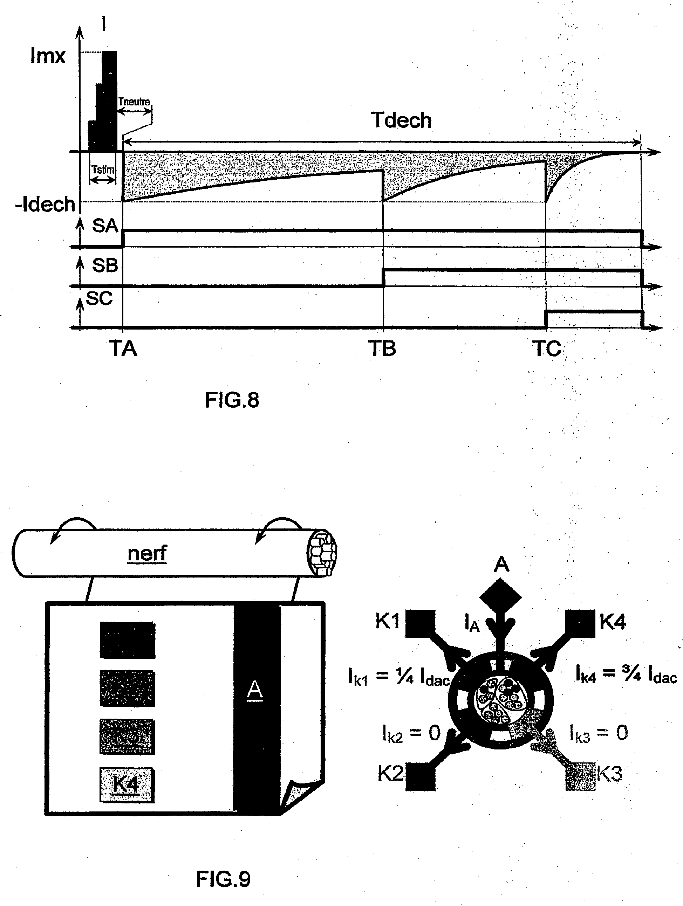

Sur la figure 8 sont illustrés des exemples non limitatifs de chronogrammes définissant les évolutions temporelles du courant I traversant l'électrode multipolaire EM (dans un souci de simplification, on suppose que l'électrode ne comporte qu'une seule cathode et que de ce fait le courant anodique est égal au courant cathodique) et des signaux de contrôle SA, SB et SC. Sur ces chronogrammes, Imx et -Idech désignent respectivement la valeur maximale et la valeur minimale du courant I, Tstim désigne la durée de stimulation, Tneutre désigne le temps séparant la phase de stimulation de la phase de décharge, Tdech désigne la durée de la phase de décharge et les instants TA, TB et TC sont les instants d'activation (mise en conduction) des transistors MiA, MiB et MiC.

Sur cette figure 8, on a représenté des niveaux logiques pour SA, SB et SC tels qu'un niveau logique « 1 » corresponde à la conduction du transistor commandé et un niveau logique « 0 » corresponde au blocage de ce transistor. Il ne s'agit donc pas de niveaux de tension. Par ailleurs, sur le chronogramme du courant I, l'échelle pour I < 0 a été volontairement dilatée afin de mieux rendre compte de l'évolution de I pendant la phase de décharge.

L'instant TA coïncide avec le début de la phase de décharge (fin du temps neutre). Les instants TB et TC, ainsi que les résistances RA et RB peuvent être, par exemple, choisis comme indiqué ci-dessous, avec Tstmx désignant la durée maximale d'une stimulation, $\alpha$ désignant la valeur maximale du rapport (Idech / Imx) et C désignant la valeur d'une capacité du réseau RCAP, et la stimulation d'amplitude maximale Imx conduisant à la plus grande quantité de charge accumulée dans la capacité étant une impulsion rectangulaire d'amplitude Imx et de durée Tstmx :

- Idech = (I x Tstmx) / (C x RA). La contrainte sur la valeur maximale de Idech conduit alors à RA = Tstmx / ($\alpha$ x C),
- TB peut être choisi tel que TB - TA = Tdech - Tstmx / $\alpha$,
- la résistance RB se déduit du choix de TB afin de respecter la contrainte sur la valeur maximale de Idech : RB = RA/(exp($\alpha$Tdech/Tstmx - 1) - 1 ),
- TC peut être choisi tel que TC - TB Tstmx(1 - exp(1 - $\alpha$Tdech/Tstmx - 1))/$\alpha$,
- RC = RA exp(1 - $\alpha$ Tdech/Tstmx)/(exp (exp($\alpha$ Tdech/Tstmx - 1) - 1) - 1).

Les valeurs de résistance ainsi obtenues correspondent à des transistors ayant un comportement d'interrupteur idéal. Par conséquent, lors de la réalisation du dispositif de contrôle de décharge DCD, il convient de retrancher de ces résistances, la résistance à l'état passant (Ron) des transistors. De plus, afin de diminuer le nombre de composants intégrés, on peut supprimer la résistance RC et dimensionner les transistors de sorte que leur résistance à l'état passant (Ron) soit égale à RC.

A titre d'exemple purement illustratif, si Tstmx = 1ms, Tdech = 20 ms, $\alpha$ = 0,1 et C = 2uF, on peut choisir RA $\approx$ 5 k$\Omega$, RB $\approx$ 3 k$\Omega$, RC $\approx$ 400 $\Omega$, TB - TA $\approx$ 10 ms et TC - TB $\approx$ 6 ms. Par ailleurs, la durée du temps neutre est faible, typiquement de l'ordre de la centaine de microsecondes.

Au moment de la stimulation, la fonction principale de l'étage de sortie ES est d'imposer sur chacune des n cathodes Ki de l'électrode multipolaire EM un courant Iki proportionnel au courant Idac que lui fournit le convertisseur numérique analogique DAC. Le rapport Iki/Idac doit par ailleurs pouvoir être choisi, pour chaque cathode Ki, par le contrôleur numérique CN via les signaux du faisceau Cfg de la figure 2.

La figure 9 illustre schématiquement, dans le cas d'une électrode multipolaire EM à 4 cathodes, enroulée sur un nerf, l'intérêt que représente le choix des rapports Iki/Idac pour sélectionner spatialement les fibres nerveuses à stimuler, et l'importance de la stabilité des rapports Iki/Idac lorsque Idac varie (les changements d'amplitude de la stimulation ne doivent en effet pas induire de changement dans la localisation spatiale de celle-ci). C'est un miroir de courant multi-sorties reconfigurable MC qui assure cette fonction principale de l'étage de sortie ES.

Un miroir de courant multi-sorties reconfigurable MC peut être constitué d'un assemblage de dispositifs élémentaires. On entend ici par « dispositif élémentaire » :

- un convertisseur tension-courant CTC, ou
- un convertisseur courant-tension CCT, ou
- un convertisseur tension-courant contrôlable CTCC, ou
- un convertisseur tension-courant à transconductance programmable CTCTP, ou
- un répartiteur de courant équilibré RCE, ou
- un répartiteur de courant équilibré contrôlable RCEC, ou
- un miroir de courant multi-sorties MCMS.

Certains de ces dispositifs élémentaires vont maintenant à être décrits au moyen d'exemples non limitatifs utilisant principalement, mais non exclusivement, des transistors à effet de champ (TEC) à canal N et à grille isolée. Des exemples similaires à base, par exemple, de transistors à effet de champ (TEC) à canal P ou de transistors bipolaires sont également envisageables.

On entend ici par « convertisseur tension-courant CTC », un dispositif électronique possédant au moins trois bornes : une borne d'entrée E, une borne de masse M et une borne de sortie S absorbant un courant Is.

On désigne ci-après par Vsm et Vem les différences de potentiels apparaissant respectivement entre la broche S et la broche M, d'une part, et la broche E et la broche M, d'autre part.

Le domaine de fonctionnement du convertisseur tension-courant CTC est défini par deux tensions Vmax et Vmin. Le convertisseur tension-courant CTC est ici considéré dans son domaine de fonctionnement si Vmin < Vsm < Vmax. Par ailleurs, dans son domaine de fonctionnement le convertisseur tension-courant CTC doit vérifier la condition Is = g(Vem) + Go Vsm, dans laquelle g() est une fonction monotone et Go est sa conductance de sortie.

Le comportement de ce convertisseur tension-courant CTC est d'autant plus satisfaisant que Go Vsm est petit devant g(Vem) et que la fonction g() s'approche d'une fonction linéaire. De plus, il est souhaitable que la résistance d'entrée vue entre les bornes E et M soit la plus grande possible.

Optionnellement, un convertisseur tension-courant CTC peut posséder des bornes d'entrée supplémentaires destinées, entre autres, à recevoir des tensions ou des courants de polarisation.

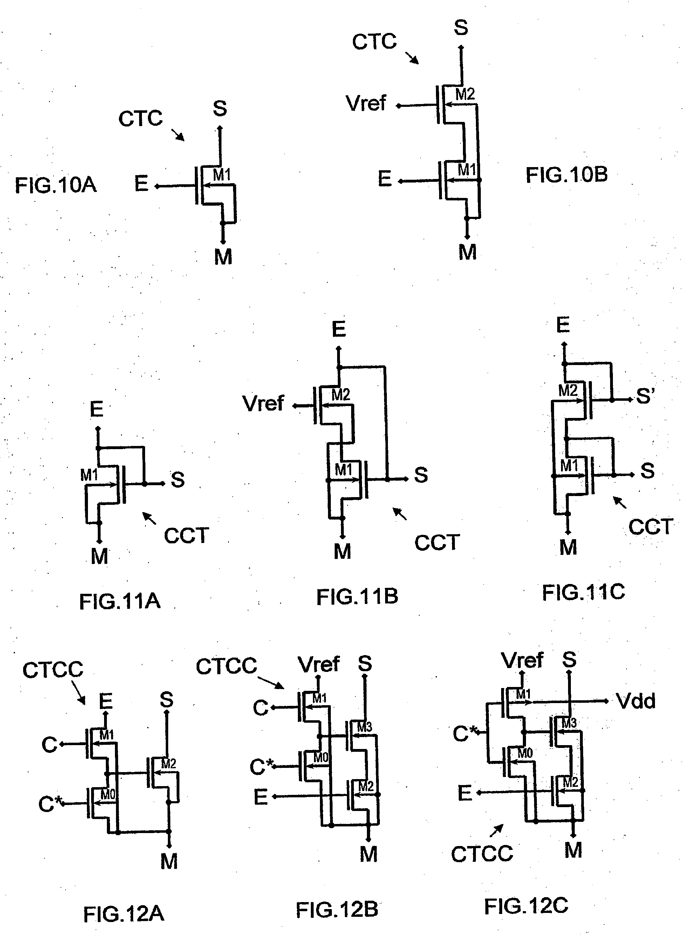

On a représenté sur les figures 10A et 10B deux exemples de réalisation non limitatifs d'un convertisseur tension-courant CTC comportant des transistors à effet de champ (TEC) à canal N et à grille isolée. Sur la figure 10B, la borne Vref représente une entrée destinée à recevoir une tension de polarisation.

On entend ici par « convertisseur courant-tension CCT », un dispositif électronique possédant au moins trois bornes : une borne d'entrée E absorbant un courant le, une borne de masse M et une borne de sortie S. La fonction principale d'un tel dispositif est d'engendrer une différence de potentiel Vsm entre sa borne S et sa borne M vérifiant la condition Vsm = f(le), où f() est une fonction monotone.

Le comportement de ce convertisseur courant-tension CCT est d'autant plus satisfaisant que la fonction f() s'approche d'une fonction linéaire. De plus, il est souhaitable que la résistance d'entrée vue entre les bornes E et M soit la plus petite possible.

Optionnellement, un convertisseur courant-tension CCT pourra posséder des bornes d'entrée supplémentaires destinées, entre autres, à recevoir des tensions ou des courants de polarisation, et/ou des bornes de sortie supplémentaires fournissant soit d'autres tensions images du courant d'entrée le, soit, plus généralement, d'autres images (tension ou courant) des grandeurs d'entrée ou de polarisation.

On a représenté sur les figures 11A à 11C trois exemples de réalisation non limitatifs d'un convertisseur courant-tension CCT comportant des transistors à effet de champ (TEC) à canal N et à grille isolée. Sur la figure 11B, la borne Vref représente une entrée destinée à recevoir une tension de polarisation. Par ailleurs, sur la figure 11C, la borne S' est une sortie supplémentaire fournissant une seconde tension image du courant d'entrée le.

Si l'on compare les figures 10 et 11, on peut remarquer qu'un convertisseur courant-tension CCT peut s'obtenir à partir d'un convertisseur tension-courant CTC auquel on applique une contre-réaction de tension.

On entend ici par « convertisseur tension-courant contrôlable CTCC », un dispositif possédant au moins quatre bornes : une borne d'entrée E, une borne de masse M, une borne de contrôle C recevant un signal logique et une borne de sortie S absorbant un courant Is.

On désigne ci-après par Vsm et Vem les différences de potentiel qui apparaissent respectivement entre la broche S et la broche M, d'une part, et la broche E et la broche M, d'autre part.

Le domaine de fonctionnement du convertisseur tension-courant contrôlable CTCC est défini par deux tensions Vmax et Vmin. Le convertisseur tension-courant contrôlable CTCC est ici considéré dans son domaine de fonctionnement lorsque Vmin < Vsm < Vmax. Par ailleurs, dans son domaine de fonctionnement le convertisseur tension-courant contrôlable CTCC doit vérifier les conditions suivantes :

- si la borne de contrôle C est au niveau logique « 0 », Is = 0 quelles que soient Vem et Vsm, et
- si la borne de contrôle C est au niveau logique « 1 », le convertisseur tension-courant contrôlable CTCC se comporte comme un convertisseur tension-courant CTC.

Optionnellement, un convertisseur tension-courant contrôlable CTCC peut également posséder des bornes d'entrée supplémentaires destinées, entre autres, à recevoir des tensions ou des courants de polarisation, et/ou une borne de contrôle complémentaire C* destinée à recevoir un signal logique complémentaire de celui reçu par la borne de contrôle C.

On a représenté sur les figures 12A à 12C trois exemples de réalisation non limitatifs d'un convertisseur tension-courant contrôlable CTCC. Sur les figures 12B et 12C, la borne Vref est une entrée destinée à recevoir une tension de polarisation. La figure 12C est identique à la figure 12B hormis le fait que le transistor M1 est maintenant un transistor à effet de champ (TEC) à canal P, ce qui permet de faire l'économie de la borne de contrôle C, mais impose que la tension Vref soit supérieure à la tension de seuil du transistor M1. La borne Vdd est par ailleurs une tension de polarisation pour le substrat du transistor M1.

Du point de vue du dimensionnement, les transistors M0 et M1, qui sont utilisés en commutation, peuvent être « taillés » au minimum autorisé par la technologie pour ce qui concerne les largeur et longueur du canal. En revanche, il est préférable de donner aux canaux des transistors M2 et M3 des longueurs et des largeurs très supérieures au minimum afin de minimiser l'influence de leur bruit et d'améliorer leur appariement entre plusieurs convertisseurs tension-courant contrôlable CTCC.

On entend ici par « convertisseur tension-courant à transconductance programmable CTCTP », un dispositif électronique possédant au moins p+3 bornes : une borne d'entrée E, une borne de masse M, une borne de sortie S absorbant un courant It et un bus de commande C[1:p] recevant des signaux logiques.

On désigne ci-après par Vsm et Vem les différences de potentiel qui apparaissent respectivement entre la broche S et la broche M, d'une part, et la broche E et la broche M, d'autre part.

Le domaine de fonctionnement du convertisseur tension-courant à transconductance programmable CTCTP est défini par deux tensions Vmax et Vmin. Le convertisseur tension-courant à transconductance programmable CTCTP est ici considéré dans son domaine de fonctionnement lorsque Vmin < Vsm < Vmax. Par ailleurs, dans son domaine de fonctionnement le convertisseur tension-courant à transconductance programmable CTCTP doit vérifier la condition It = N g(Vem) + Go Vsm, où N est la valeur numérique codée par le bus de commande, g() est une fonction monotone et Go est la conductance de sortie du convertisseur tension-courant à transconductance programmable CTCTP, laquelle est éventuellement une fonction de n. Plusieurs codages sont envisageables, et notamment le code binaire naturel ou bien un code non minimal comme par exemple le nombre de signaux portés à l'état logique « 1 ».

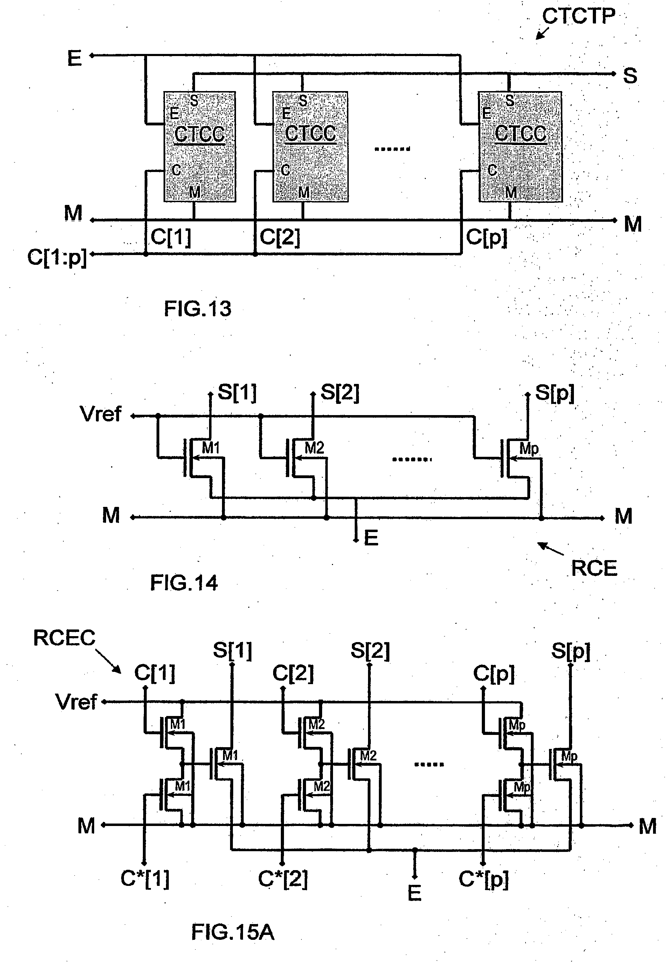

Comme cela est schématiquement illustré sur la figure 13, un convertisseur tension-courant à transconductance programmable CTCTP peut être réalisé en associant p convertisseurs tension-courant contrôlable CTCC comme indiqué ci-dessous :

- la borne d'entrée E de chacun des p convertisseurs tension-courant contrôlables CTCC est reliée à la borne d'entrée E du convertisseur tension-courant à transconductance programmable CTCTP,
- la borne de sortie S de chacun des p convertisseurs tension-courant contrôlables CTCC est reliée à la borne de sortie S du convertisseur tension-courant à transconductance programmable CTCTP (le courant It est alors égal à la somme des courants Is absorbés par chacun des convertisseurs tension-courant contrôlables CTCC,
- la borne de masse M de chacun des convertisseurs tension-courant contrôlables CTCC est reliée à la borne de masse M du convertisseur tension-courant à transconductance programmable CTCTP,
- la borne de commande de chacun des convertisseurs tension-courant contrôlables CTCC est reliée à exactement un signal du bus de commande C[1:p] du convertisseur tension-courant à transconductance programmable CTCTP,
- si les convertisseurs tension-courant contrôlables CTCC possèdent des bornes d'entrée supplémentaires, celles-ci seront câblées de telle manière que tous les convertisseurs tension-courant contrôlables CTCC aient le même comportement,
- si les convertisseurs tension-courant contrôlables CTCC possèdent une borne de contrôle complémentaire C*, alors le convertisseur tension-courant à transconductance programmable CTCTP doit posséder un bus de commande complémentaire C*[1:p], et
- le code utilisé pour l'encodage de la valeur numérique d'entrée du convertisseur tension-courant à transconductance programmable CTCTP est le nombre de signaux portés à l'état logique « 1 » dans le bus de commande.

Il est également possible de réaliser un convertisseur tension-courant à transconductance programmable CTCTP utilisant le code binaire naturel avec $(2^p - 1)$ convertisseurs tension-courant contrôlables CTCC. Dans ce cas, le signal C[1] est connecté à un seul convertisseur tension-courant contrôlable CTCC, le signal C[2] est connecté à exactement deux convertisseurs tension-courant contrôlables CTCC, le signal C[3] est connecté à exactement quatre convertisseurs tension-courant contrôlables CTCC, et ainsi de suite jusqu'au signal C[p] qui est connecté à 2$^p$-1 convertisseurs tension-courant contrôlables CTCC.

On entend ici par « répartiteur de courant équilibré RCE » un dispositif à p+1 bornes possédant : une borne d'entrée E fournissant un courant le et un bus de sorties S[1:p] absorbant chacune un courant ISi. Dans ce qui suit, d'autres bornes supplémentaires que celles présentées peuvent être envisagées afin de fournir au dispositif des tensions ou des courants de polarisation ainsi que la masse.

On désigne ci-après par VSi la différence de potentiel entre la borne de sortie S[i] et la borne d'entrée E.

Le domaine (convexe) de tensions de fonctionnement du répartiteur de courant équilibré RCE est défini par Vmin < VSi < Vmax (Vmin et Vmax étant deux tensions de même signe vérifiant Vmin < Vmax) quelque soit i, entier, appartenant à l'intervalle [1, p]. Par ailleurs, le comportement du répartiteur de courant équilibré RCE est défini par la relation ISi = le/p, quel que soit I, entier appartenant à l'intervalle [1, p].

Afin que le fonctionnement de ce répartiteur soit satisfaisant, il est nécessaire qu'il présente une résistance d'entrée la plus faible possible et des résistances de sortie les plus grandes possibles.

On a représenté schématiquement sur la figure 14, à titre non limitatif, un exemple de réalisation d'un répartiteur de courant équilibré RCE comportant des transistors à effet de champ à canal N.

En ce qui concerne le dimensionnement, les transistors Mi utilisés possèdent tous une même largeur et une même longueur, lesquelles sont préférentiellement non minimales afin d'améliorer l'appariement des transistors.

Sur la figure 14, Vref représente une tension de référence et M représente une borne de masse correspondant à la polarisation du substrat des transistors.

On entend ici par « répartiteur de courant équilibré contrôlable RCEC », un dispositif à 2p+1 bornes possédant : une borne d'entrée E fournissant un courant le, un bus de sorties Sr[1:p] absorbant chacune un courant ISi et un bus de commande C[1:p] recevant des signaux logiques.

On désigne ci-après par VSi la différence de potentiel entre la borne de sortie S[i] et la borne d'entrée E, et par N le nombre d'entrées de commande recevant un signal logique à « 1 ».

Le domaine (convexe) de tensions de fonctionnement du répartiteur de courant équilibré contrôlable RCEC est défini par Vmin < VSi < Vmax (Vmin et Vmax étant deux tensions de même signe vérifiant Vmin < Vmax) quel que soit i, entier appartenant à l'intervalle [1, p]. Par ailleurs, le comportement du répartiteur de courant équilibré contrôlable RCEC est défini par les conditions suivantes :

- si C[i] est au niveau logique « 0 », alors ISi = 0, et
- si C[i] est au niveau logique « 1 », alors ISi = le/N.

Optionnellement, un répartiteur de courant équilibré contrôlable RCEC peut également posséder des bornes d'entrée supplémentaires destinées, entre autres, à recevoir des tensions ou des courants de polarisation, et/ou un bus de contrôle complémentaire C*[1:p] destiné à recevoir des signaux logiques complémentaires de ceux reçus par le bus C[1:p].

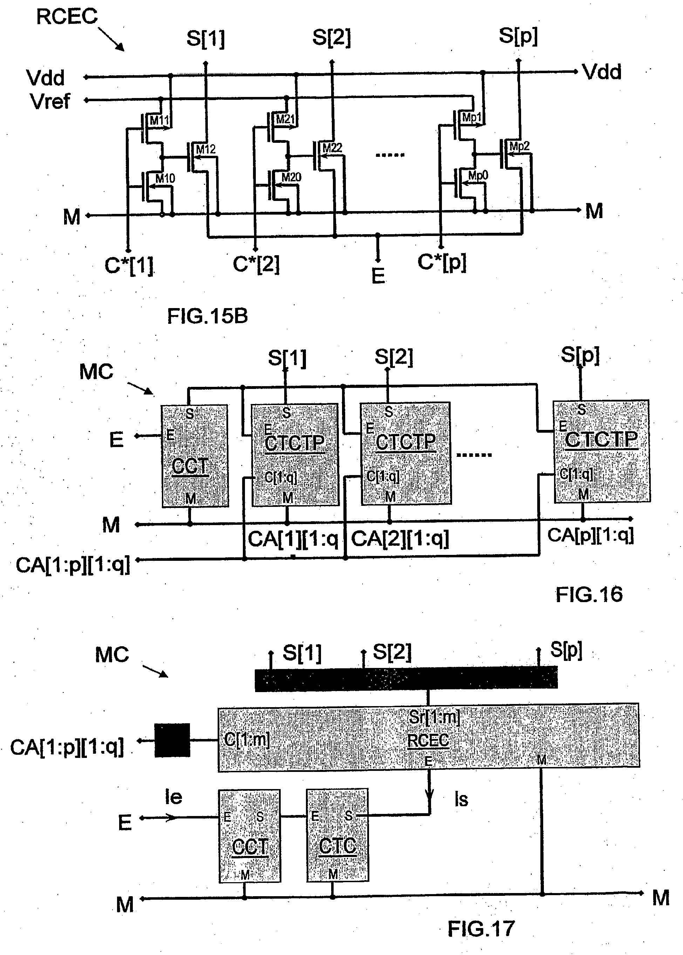

On a représenté schématiquement sur les figures 15A et 15B, à titre non limitatif, deux exemples de réalisation d'un répartiteur de courant équilibré contrôlable RCEC comportant des transistors à effet de champ. L'exemple illustré sur la figure 15A n'utilise que des transistors à canal N. Il nécessite 2p entrées de contrôle (C[1:p] et C*[1:p]) et la tension Vref est limitée à Vdd moins une tension de seuil de transistor à canal N (Vdd étant la tension correspondant à un niveau logique haut sur les entrées de contrôle).

L'exemple illustré sur la figure 15B utilise des transistors à canal N et des transistors à canal P. Il ne nécessite plus que p entrées de contrôle. Il est cependant nécessaire d'ajouter une tension de polarisation (Vdd) pour les substrats des transistors à canal P. De plus, le fonctionnement du dispositif n'est assuré que pour une tension Vref supérieure à une tension de seuil de transistor à canal P. Au moyen de ces deux exemples, on peut construire une autre variante, moins contraignante au niveau de l'excursion de Vref, en remplaçant les transistors Mi1 par des portes de transmission CMOS constituées d'un transistor à canal N et d'un transistor à canal P, mis en parallèle au niveau de leurs drains et sources et commandés par des signaux complémentaires au niveau de leurs grilles.

En ce qui concerne le dimensionnement, les transistors Mi0 et Mi1, utilisés en commutation, sont au minimum autorisé par la technologie, tandis que les transistors Mi2 ont tous une même largeur et une même longueur, lesquelles sont de préférence non minimales de manière à améliorer l'apparierilent des transistors.

Sur les figures 15A et 15B, Vref représente une tension de référence et M représente une borne de masse correspondant à la polarisation du substrat des transistors à canal N, et Vdd sert à la polarisation des substrats des transistors à canal P.

On entend ici par « miroir de courant multi-sorties MCMS », un dispositif électronique à p+2 bornes possédant : une borne de masse M, une borne d'entrée E et un bus de sorties S[1:p]. Dans ce qui suit, d'autres bornes supplémentaires que celles présentées peuvent être envisagées afin de fournir au dispositif des tensions ou des courants de polarisation, notamment.

On désigne ci-après par le le courant absorbé par la borne d'entrée E, ISi le courant absorbé par la borne de sortie S[i] et VSi la différence de potentiel entre S[i] et M.

Le domaine (convexe) de tensions de fonctionnement du miroir de courant multi-sorties MCMS est défini par Vmin < VSi < Vmax (Vmin et Vmax étant deux tensions de même signe vérifiant Vmin < Vmax) quel que soit i, entier appartenant à l'intervalle [1,p]. Par ailleurs, le comportement du miroir de courant multi-sorties MCMS est défini par la condition ISi = Ai le + Gi VSi + {somme de j = 1 à p des Gij (VSi - VSj)}, où Ai représente l'amplification en courant de la branche i du miroir de courant multi-sorties MCMS, Gi représente la conductance de sortie de la branche i du miroir de courant multi-sorties MCMS et Gij représente la conductance différentielle de sortie entre les branches i et j du miroir de courant multi-sorties MCMS.

On entend ici par « miroir de courant multi-sorties reconfigurable MC », un miroir de courant multi-sorties MCMS pour lequel les amplifications en courant de chaque branche (Ai) peuvent être choisies en cours de fonctionnement.

Un tel miroir est un dispositif électronique à (p (q + 1) + 2) bornes possédant : une borne de masse M, une borne d'entrée E, un bus de sorties S[1:p] et un bus de commandes CA[1:p][1:q]. Dans ce qui suit, d'autres bornes supplémentaires que celles présentées peuvent être envisagées afin de fournir au dispositif des tensions ou des courants de polarisation, notamment.

Chaque sous-bus de commande CA[i][1:q] règle l'amplification en courant d'une seule branche (sortie) du miroir de courant multi-sorties reconfigurable MC. Le codage de ce sous-bus ainsi que son influence sur l'amplification Ai dépendent du mode de réalisation du miroir de courant multi-sorties reconfigurable MC.

Deux classes de miroir de courant multi-sorties reconfigurable MC peuvent être définies : la classe des miroirs de courant multi-sorties reconfigurables modulaires et celle des miroirs de courant multi-sorties reconfigurables à répartiteur.

Comme cela est schématiquement illustré sur la figure 16, un miroir de courant multi-sorties reconfigurable modulaire est constitué par association d'un convertisseur courant-tension CCT, tel que défini ci-avant, avec p convertisseurs tension-courant à transconductance programmable CTCTP, tels que défini ci-avant.

Cette association peut se faire de la manière suivante :

- la borne d'entrée E du convertisseur courant-tension CCT est reliée à la borne d'entrée E du miroir de courant multi-sorties reconfigurable modulaire MC,
- la borne de masse M du convertisseur courant-tension CCT ainsi que les bornes de masse M des p convertisseurs tension-courant à transconductance programmable CTCTP sont reliées à la borne de masse M du miroir de courant multi-sorties reconfigurable modulaire MC,
- la borne d'entrée E de chacun des convertisseurs tension-courant à transconductance programmable CTCTP est reliée à la borne de sortie S du convertisseur courant-tension CCT,
- la borne de sortie S de chacun des p convertisseurs tension-courant à transconductance programmable CTCTP est reliée à exactement une sortie S[i] du miroir de courant multi-sorties reconfigurable modulaire MC, et
- chaque sous-bus de commande CA[i][1:p] du miroir de courant multi-sorties reconfigurable modulaire MC est relié au bus de commande C[1:p] du convertisseur tension-courant à transconductance programmable CTCTP dont la sortie S est reliée à la sortie S[i] du miroir de courant multi-sorties reconfigurable modulaire MC.

Le codage des sous-bus de commande CA[i][1:q] est alors choisi par l'architecture du convertisseur tension-courant à transconductance programmable CTCTP, comme indiqué ci-dessous :

- soit Iti le courant absorbé par la borne de sortie S[i],
- soit le le courant absorbé par la borne d'entrée E (du miroir de courant multi-sorties reconfigurable modulaire MC),
- soit VSi la différence de potentiel entre la borne S[i] et la borne M,
- soit Ni la valeur numérique codée par le sous-bus de commande CA[i][1:q] (comme indiqué dans la définition précédente du convertisseur tension-courant à transconductance programmable CTCTP),
- soit Vs = f (le) la relation caractérisant la transrésistance du convertisseur courant-tension CCT,
- soit It = N g (Vem) + Go Vsm la relation caractérisant la transconductance d'un convertisseur tension-courant à transconductance programmable CTCTP.

Par ailleurs, le comportement du miroir de courant multi-sorties reconfigurable modulaire MC est décrit par la relation Iti = Ni g(f(le)) + Gi VSi + {somme de j = 1 à p des Gij (VSi - VSj)}.

En comparant cette expression avec celle qui définit le comportement d'un miroir de courant multi-sorties MCMS, il apparaît que les fonctions f et g doivent être telles que leur composition donne une fonction linéaire, au moins pour la plage de courant d'entrée utile. Par conséquent, l'architecture du convertisseur courant-tension CCT et celle des convertisseurs tension-courant à transconductance programmable CTCTP doivent être appariées. Pour ce faire, on peut utiliser, par exemple, un convertisseur courant-tension CCT du type de celui illustré sur la figure 11A avec un convertisseur tension-courant à transconductance programmable CTCTP réalisé par association de convertisseurs tension-courant contrôlables CTCC du type de celui illustré sur la figure 12A. Par exemple, le convertisseur courant-tension CCT illustré sur la figure 11B peut être utilisé indifféremment avec les convertisseurs tension-courant contrôlables CTCC illustrés sur les figures 12B et 12C.

Comme cela est schématiquement illustré sur la figure 17, un miroir de courant multi-sorties reconfigurable à répartiteur est réalisé en associant un miroir de courant, constitué d'un convertisseur courant-tension CCT et d'un convertisseur tension-courant CTC, à un répartiteur de courant équilibré contrôlable RCEC à m sorties.

Cette association peut se faire de la manière suivante :

- la borne d'entrée E du convertisseur courant-tension CCT est reliée à la borne d'entrée E du miroir de courant multi-sorties reconfigurable à répartiteur MC, et le est le courant absorbé par cette borne,
- la borne de masse M du convertisseur courant-tension CCT ainsi que les bornes de masse M du convertisseur tension-courant CTC et du répartiteur de courant équilibré contrôlable RCEC sont reliées à la borne de masse M du miroir de courant multi-sorties reconfigurable à répartiteur MC,
- la borne d'entrée E du répartiteur de courant équilibré contrôlable RCEC est reliée à la borne S du convertisseur tension-courant CTC, et Is est le courant absorbé par le convertisseur tension-courant CTC sur sa borne S,
- le bus de commande du miroir de courant multi-sorties reconfigurable à répartiteur MC CA[1:p][1:q] est relié, d'une façon qui sera précisée ultérieurement, au bus de commande C[1:m] du répartiteur de courant équilibré contrôlable RCEC, et
- le bus de sortie S[1:p] du miroir de courant multi-sorties reconfigurable à répartiteur MC est relié, d'une façon qui sera précisée ultérieurement, au bus de sorties Sr[1:m] du répartiteur de courant équilibré contrôlable RCEC.

Pour ce qui concerne l'association du convertisseur tension-courant CTC et du répartiteur de courant équilibré contrôlable RCEC, en désignant par N le nombre de signaux portés à l'état logique '1' dans le bus C[1:m], Isri le courant absorbé par la sortie Sr[i] du répartiteur de courant équilibré contrôlable RCEC, Vs = f (le) la relation caractérisant la transrésistance du convertisseur courant-tension CCT, et Is = g (Vem) la relation caractérisant la transconductance du convertisseur tension-courant CTC, et en négligeant les effets des conductances de sortie, nécessairement finies, on a les relations suivantes :

$$Is = g\ (f(Ie)) \qquad\qquad (1)$$

$$Is = \{\text{somme de } j = 1 \text{ à } m \text{ des } Isri\} \quad (2)$$

$$Isri = Is\ /\ N \text{ si } Cr[i] = \text{'1', sinon } 0 \quad (3)$$

En comparant ces expressions avec celles qui définissent le comportement d'un miroir de courant multi-sorties reconfigurable, on peut noter que les fonctions f et g doivent être telles que leur composition donne une fonction linéaire, au moins pour la plage de courant d'entrée utile. Par conséquent, les architectures du convertisseur courant-tension CCT et du convertisseur tension-courant CTC doivent être appariées. Pour ce faire, on peut utiliser, par exemple, un convertisseur tension-courant CTC du type de celui illustré sur la figure 10A avec un convertisseur courant-tension CCT du type de celui illustré sur la figure 11A. Par exemple, le convertisseur tension-courant CTC illustré sur la figure 10B peut être utilisé indifféremment avec les convertisseurs courant-tension CCT illustrés sur les figures 11B et 11C.

On peut également noter que la somme des courants de sortie du miroir de courant multi-sorties reconfigurable à répartiteur MC n'est pas configurable. Elle ne dépend que du courant d'entrée et de l'amplification en courant du miroir élémentaire constitué par l'association du convertisseur tension-courant CTC et du convertisseur courant-tension CCT.

En outre, on peut également noter que les interconnexions des bus de commandes et des bus de sorties dépendent du codage adopté pour la représentation des valeurs numériques sur les sous-bus de commandes CA[i][1:q]. Deux exemples purement illustratifs et non limitatifs de miroir de courant multi-sorties reconfigurable, à répartiteur MC pour un codage du type « nombre de signaux à '1' » et pour un codage binaire naturel sont donnés ci-après.

En remplaçant la relation (1) Is = g(f(le)) par la relation Is = A le et en appelant Ni la valeur numérique codée par le sous-bus de commande CA[i][1:q], on obtient la relation ISi = Ni A le / {somme de j=1 à p des Ni} qui définit le fonctionnement d'un miroir de courant multi-sorties reconfigurable à répartiteur MC.

Pour réaliser un miroir de courant multi-sorties reconfigurable à répartiteur MC pour un codage du type « nombre de signaux à '1' », on utilise un répartiteur de courant équilibré contrôlable RCEC à m = pq sorties. Chacune des p sorties S[i] du miroir de courant multi-sorties reconfigurable à répartiteur MC doit être connectée à exactement q sorties du répartiteur de courant équilibré contrôlable RCEC. De plus, les q signaux du sous-bus CA[i][1:q] doivent être connectés aux q signaux de commande du répartiteur de courant équilibré contrôlable RCEC qui contrôlent les sorties connectés à S[i]. On peut, par exemple, réaliser un premier ensemble de connexions dans lequel tous les signaux du segment de bus Sr[(i-1)q + 1 : iq ] sont connectés à S[i] et un second ensemble de connexions dans lequel le segment de bus C[(i-1)q + 1 : iq] est connecté au sous-bus CA[i][1:q].

Pour réaliser un miroir de courant multi-sorties reconfigurable à répartiteur MC pour un codage binaire naturel, on utilise un répartiteur de courant équilibré contrôlable RCEC à m = p(2$^q$ - 1) sorties. Chacune des p sorties S[i] du miroir de courant multi-sorties reconfigurable à répartiteur MC doit être connectée à exactement (2$^q$ - 1) sorties du répartiteur de courant équilibré contrôlable RCEC. De plus, les q signaux du sous-bus CA[i][1:q] doivent être connectés aux (2$^q$ - 1) signaux de commande du répartiteur de courant équilibré contrôlable RCEC qui contrôlent les sorties connectées à S[i] : CA[i][1] connecté à un signal de commande, CA[i][2] connecté à deux signaux de commande, CA[i][3] connecté à 4 signaux de commande et, plus généralement, CA[i][j] connecté à 2$^{(j-1)}$ signaux de commande. On peut, par exemple, réaliser un premier ensemble de connexions dans lequel tous les signaux du segment de bus Sr[(i - 1)(2$^q$ - 1) + 1 : i (2$^q$ - 1)] sont connectés à S[i] et un second ensemble de connexions dans lequel tous les signaux du segment de bus C[(i - 1)(2$^q$ - 1) + 2 $^{(j-1)}$ : (i - 1)(2$^q$ - 1) + 2$^j$ - 1] sont connectés au signal CA[i][j]. CA[i][q] désigne ici le bit de poids fort de la représentation binaire.

Au moment de la stimulation, la fonction principale de l'étage de sortie ES est d'imposer sur chacune des n cathodes Ki de l'électrode multipolaire EM un courant Iki proportionnel au courant Idac que lui fournit le convertisseur numérique analogique DAC. De plus, le rapport Iki / Idac doit pouvoir être choisi, pour chaque cathode Ki, par le contrôleur numérique CN via les signaux du faisceau Cfg représenté sur la figure 2. Dans l'exemple illustré, n = 4 (i = 1 à 4), mais, comme indiqué précédemment il peut prendre n'importe quelle valeur supérieure ou égale à 2.

D'une manière générale, le miroir de courant MC de l'invention peut être réalisé avec un miroir de courant multi-sorties reconfigurable à n sorties. Comme cela est illustré sur la figure 6, le miroir de courant MC est interconnecté aux autres éléments de l'étage de sortie ES par son bus de commandes CA qui constitue le faisceau Cfg, son entrée E étant connectée au signal Idac et ses sorties S[1:n] étant respectivement connectées aux signaux K'i.

Le courant Ist, qui circule dans l'anode A de l'électrode multipolaire EM, représente la somme des courants Iki circulant dans les différentes cathodes Ki. Il est important de noter que ce courant Ist n'est pas forcément réparti de façon égale entre les n différentes cathodes Ki, afin que chacune d'entre elles dispose d'un courant égal à Ist/n. Il doit être en effet possible de répartir ce courant Ist de façon inégale entre les n différentes cathodes Ki, ou bien seulement entre certaines d'entre elles.

Par exemple, en présence de quatre cathodes K1 à K4 (n=4), on peut avoir des répartitions de type (1/4,1/4,1/4,1/4), ou (1/4,1/4,1/2,0), ou encore (1/4,0,0,3/4), ou encore (1/3,1/3,1/3,0), ou encore (0,1/3,0,2/3), ou encore (1/5,1/5,2/5,1/5), ou encore (2/5,0,3/5,0), ou encore (1/6,1/6,2/6,2/6), ainsi que toutes les permutations possibles. Ces différentes répartitions permettent de contrôler la localisation spatiale de la stimulation dans le nerf.

Afin de séparer les commandes permettant de localiser la stimulation de celles contrôlant son amplitude, on peut imposer que le rapport Ist / Idac ne soit pas configurable, c'est-à-dire qu'un changement de répartition ne modifie pas l'amplitude de l'impulsion globale de stimulation (mesurée à l'anode A de l'électrode multipolaire EM). C'est le contrôleur numérique CN qui, par les commandes qu'il applique au miroir de courant MC, impose les répartitions de courant. Ces commandes lui servent aussi à spécifier les instants de début et de fin de stimulation.

A titre d'exemple, on décrit ci-après un miroir de courant MC destiné à être utilisé avec une électrode multipolaire EM à quatre cathodes K1 à K4 et pour des répartitions de courant consistant en des combinaisons des valeurs {0, ¼, 1/3, ½, 2/3, ¾, 1} prises 4 à 4 et telles que la somme des éléments de chaque combinaison soit égale à l'unité. De plus, on impose d'avoir un rapport Ist / Idac égal à quatre.

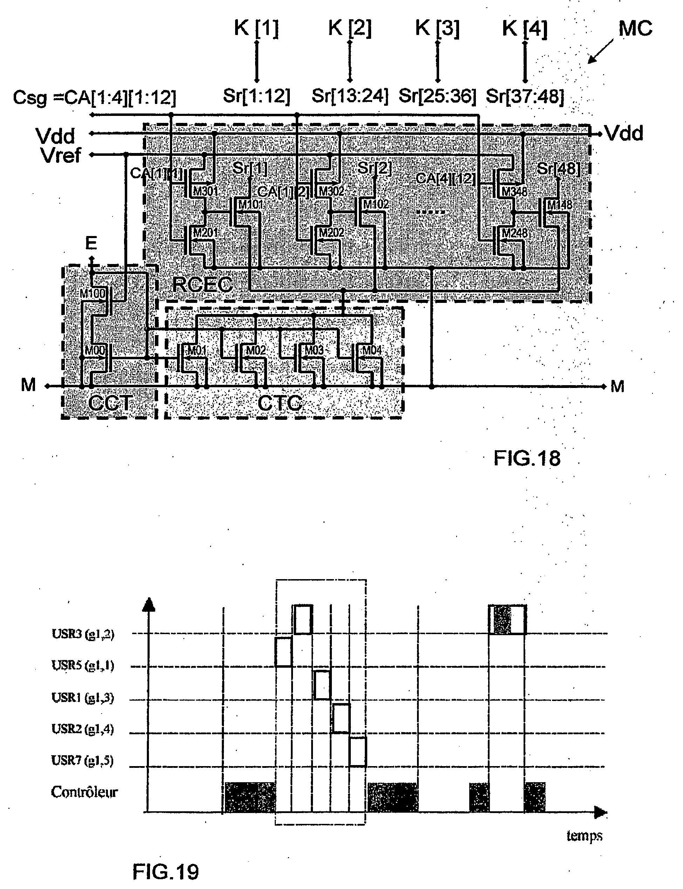

En présence de la contrainte sur la stabilité du rapport Iki. / Idac, il est préférable d'utiliser un miroir de courant multi-sorties reconfigurable à répartiteur MC, du type de celui illustré sur la figure 18.

Dans cet exemple, le rapport Ist / Idac égal à quatre est obtenu en imposant que les transistors M00, M01, M02, M03 et M04 aient tous les mêmes dimensions (et qu'ils soient dessinés et placés sur le substrat dans les règles de l'art de manière à maximiser leur appariement).

Le nombre m de sorties du répartiteur de courant équilibré contrôlable RCEC est déterminé en considérant qu'il doit toujours avoir le même nombre r de sorties actives afin d'imposer toujours la même tension de sortie au convertisseur tension-courant CTC pour assurer une meilleure stabilité au courant Ist lors des changements de répartition. Le plus petit commun multiple de 4, 3 et 2 étant 12, il faut par conséquent choisir un répartiteur de courant équilibré contrôlable RCEC comportant 4*12 = 48 sorties, commandé de manière à avoir en permanence 12 sorties actives. On peut noter en passant que ce choix permet d'élargir la gamme de valeurs utilisables dans les répartitions. Celle-ci devient en effet {0, 1/6, ¼, 1/3, ½, 2/3, ¾, 5/6, 1}.

Le faisceau Cfg issu du contrôleur numérique CN est donc constitué de 48 signaux logiques organisés en 4 sous-bus de 12 signaux chacun. Le nombre de signaux portés à l'état logique « 1 » (actifs) sur un sous-bus représente le nombre de douzièmes du courant total de stimulation qui sont appliqués sur la cathode correspondante. Ceci n'est cependant vrai que si le nombre total de signaux actifs sur le faisceau Cfg est exactement égal à 12.

Le contrôleur numérique CN peut également utiliser les signaux du faisceau Cfg pour bloquer les cathodes Ki en dehors des instants de stimulation.

Le répartiteur de courant équilibré contrôlable RCEC illustré sur la figure 18 est sensiblement identique à celui décrit précédemment en référence à la figure 15B. Sa tension Vref ne peut donc pas être inférieure à une tension de seuil de transistor à canal P. Cependant, on peut utiliser à la place un répartiteur RCEC du type de celui décrit précédemment en référence à la figure 15A, mais dans ce cas le bus Cfg doit porter 96 signaux à moins de compléter les signaux au niveau du miroir de courant MC. Il est même envisageable de délocaliser une partie des fonctions du contrôleur numérique CN au niveau du miroir de courant MC et de ne plus transmettre que des instructions codées sur le faisceau Cfg.

Afin d'améliorer l'appariement du convertisseur courant-tension CCT et du convertisseur tension-courant CTC, on utilise de préférence la même tension de référence Vref pour le convertisseur courant-tension CCT et pour le répartiteur de courant équilibré contrôlable RCEC. Par ailleurs, pour que les transistors M0i soient placés dans des conditions les plus similaires possibles pour ce qui concerne leur différence de potentiel drain-source, le transistor M100 est traversé par le courant Idac alors que douze transistors du répartiteur de courant équilibré contrôlable RCEC sont traversés par un courant égal à 4*Idac. Par conséquent, le transistor M100 doit avoir une longueur de canal identique à celle des transistors M101 à M148 et une largeur de canal égale à trois fois celle desdits transistors M101 à M148.

Cette structure à répartiteur présente une valeur de conductance de sortie inter-électrode qui est relativement importante (alors que la conductance de sortie de mode commun est très faible grâce à la structure cascode implicite de ce dispositif). Pour remédier à cet inconvénient, on peut, dans une variante, utiliser un miroir de courant multi-sorties reconfigurable modulaire MC comportant quatre convertisseurs tension-courant à transconductance programmable CTCTP à 12 entrées. La séparation des miroirs de courant élémentaires permet de faire disparaître complètement la conductance de sortie inter-électrode, au prix d'une légère augmentation de la conductance de mode commun et d'un risque un peu plus élevé de dispersion des caractéristiques d'une sortie à l'autre.

Comme évoqué précédemment, l'invention propose également un protocole de communication adapté aux transmissions sans fil entre le contrôleur CR et les implants I (ou unités de stimulation répartie USRs), du type de celui décrit ci-avant, d'une installation de stimulation.

Bien entendu, l'invention n'est pas limitée aux seules transmissions par voie d'ondes entre le contrôleur CR et les implants I (ou USR). On peut en effet envisager un mode de transmission sur bus filaire sans que le protocole proposé ne perde sa pertinence.

En présence de transmissions sans-fil (c'est-à-dire par voie d'ondes), l'acquittement est l'unique moyen de s'assurer de la bonne réception d'une trame de paquets de données. Par ailleurs, le mode d'accès au médium est important. La méthode d'accès au médium et le modèle de coopération entre les différentes entités, constituant l'installation de stimulation, sont étroitement liés. Les collisions ne sont en revanche pas nécessairement détectées.

Préférentiellement, la gestion des liaisons logiques entre les entités physiquement en relation se fait sans connexion, avec acquittement sur demande et sans contrôle de flux.

En raison du type du milieu considéré dans l'exemple décrit (intracorporel), un compromis doit être trouvé entre la fiabilité lors de l'échange des trames, et la complexité des transmissions.

Il est tout d'abord nécessaire, dans certains cas, de s'assurer qu'une trame envoyée a bien été reçue, voire même qu'une opération ordonnée par le contrôleur CR (dans une requête transmise) a bien été exécutée, en particulier lorsque le corps stimulé est celui d'un humain.

A cet effet, il est avantageux de permettre au contrôleur CR de demander un acquittement dans certaines situations précises (c'est-à-dire de façon non systématique). Par exemple, on peut prévoir qu'aucun échange de données ne peut se poursuivre tant qu'un acquittement n'a pas été reçu par le contrôleur CR. Cela permet d'éviter un contrôle de flux.

L'établissement d'une connexion permet, d'une manière générale, de garantir que les noeuds (ou entités concernées) sont actifs et qu'ils sont en mesure de participer à la communication. Préférentiellement les noeuds de l'installation sont toujours dans l'attente d'une réception de trame.

Lorsque les collisions ne sont pas détectées, il est important de les éviter ou d'en minimiser les risques.

Plusieurs méthodes d'accès peuvent être envisagées.

Une première méthode, dite statique, consiste à imposer à chaque émetteur un intervalle de temps pendant lequel il est autorisé à émettre. Cela peut se faire par un multiplexage temporel. L'accès est alors de type déterministe.

Cette méthode statique n'offre pas une exploitation performante du médium et amène à considérer systématiquement tous les noeuds, même ceux « inactifs ».

Une deuxième méthode, dite par compétition, consiste à permettre à chaque émetteur (ou unité de stimulation répartie USR) d'émettre lorsqu'il en a besoin, indépendamment des autres. Si deux émetteurs émettent en même temps, un conflit survient et une procédure spéciale doit être mise en oeuvre pour régler le conflit.

Avec ce type de méthode, il est indispensable de limiter le risque de collision par la maîtrise du droit de parole (DDP) des noeuds du réseau. La réaction à d'éventuelles erreurs est importante mais il faut cependant minimiser les risques de collisions afin d'éviter la non-réception des messages importants. Par conséquent, une gestion du droit de parole est nécessaire.

Une troisième méthode, dite par élection, consiste à choisir dynamiquement l'émetteur. Dans le cas d'une gestion centralisée un noeud maître est chargé de prendre les décisions, tandis que dans le cas d'une gestion distribuée des jetons sont échangés entre les noeuds. L'accès est alors de type probabiliste.

La gestion centralisée pose le problème du droit de libre parole des esclaves, c'est-à-dire des différentes unités de stimulation réparties (ou USRs). Bien qu'elle soit assez sûre, la scrutation systématique de toutes les USRs n'est pas très efficace étant donné qu'elle induit des échanges inutiles avec les noeuds inactifs et pénalise la réactivité de l'installation. En effet, toutes les USRs ne sont pas forcément impliquées dans toutes les phases d'un mouvement. Par conséquent, dans une configuration donnée, et pour une phase donnée, seules les USRs d'un sous-ensemble doivent être actives et donc autorisées à s'exprimer, par exemple pour notifier un problème tel qu'une erreur de stimulation.

Cette troisième méthode étant celle qui est préférée, elle va maintenant être décrite plus en détails.

Il est tout d'abord nécessaire de différencier l'attribution d'un droit de parole à un noeud (DDP individuel) de l'attribution d'un droit de parole à un groupe de noeuds (DDP groupe).

Il est rappelé qu'un noeud esclave (ici une USR) dispose automatiquement du droit de parole dès qu'on lui demande d'acquitter. L'USR peut donc profiter de l'occasion pour signaler la détection d'une erreur (antérieure ou relative à l'exécution d'une opération en cours). Il appartient alors au maître (le contrôleur CR) de permettre à l'USR de décrire l'erreur (c'est-à-dire d'envoyer un vecteur descriptif de(s) l'erreur(s) détectée(s)). Cette permission se fait au moyen d'un DDP individuel.

Le DDP de groupe est notamment destiné à permettre aux USRs actives de signaler une erreur sans être observées. Plus précisément, on attribue à chaque groupe un DDP de groupe pendant une fenêtre temporelle choisie, de sorte que les USRs de chaque groupe puissent émettre si elles l'estiment nécessaire.

Préférentiellement, pour limiter le risque de collision à l'intérieur d'une fenêtre temporelle, on alloue un intervalle de temps à chaque USR. Chaque USR se positionne temporellement, automatiquement, dès que le contrôleur CR lui a indiqué qu'il a attribué un DDP au groupe auquel il appartient. Bien entendu, chaque USR connait son groupe d'appartenance ainsi que sa position au sein du groupe, laquelle est définie par un niveau de priorité qui peut différer d'un groupe à l'autre et qui définit la position de l'intervalle de temps qui lui est alloué au sein de la fenêtre temporelle de parole du groupe.

La durée des intervalles temporels dépend de la technologie de communication retenue (caractéristiques de la transmission en terme de débit, précision de la synchronisation des USRs concernées (retard, gigue), etc).

Sur la figure 19 se trouvent illustrés, d'une part à droite, un exemple d'attribution et d'utilisation d'un DDP individuel par une USR (ici l'USR3), et d'autre part à gauche, un exemple d'attribution d'un DDP de groupe. Le groupe est, dans cet exemple, constitué de 5 USRs dont l'ordre de priorité est par exemple USR5, USR3, USR1, USR2, USR7.

Chaque USR se positionne selon l'intervalle temporel D qui lui a été alloué dans la fenêtre temporelle allouée à son groupe. Ce positionnement est défini par la durée de l'intervalle temporel D et le niveau de priorité (et donc la position) de l'USR. La date de début de l'intervalle temporel est D*. Les petits cadres rectangulaires noirs représentent les DDPs individuels, le cadre rectangulaire en tiretés-points représente le DDP du groupe, les rectangles gris représentent les accès au médium par le contrôleur CR, et chaque référence USRi(g,k) représente l'USR numéro i, appartenant au groupe g et disposant d'un niveau de priorité k dans le groupe g.

Il est important de noter qu'une émission n'est possible qu'à condition que le médium soit libre. Par ailleurs, il est également important de noter que le positionnement d'une USR est relatif. En effet, chaque USR calcule la date de début de son intervalle temporel par rapport à l'instant de réception du message d'attribution du DDP de groupe envoyé par le contrôleur CR.

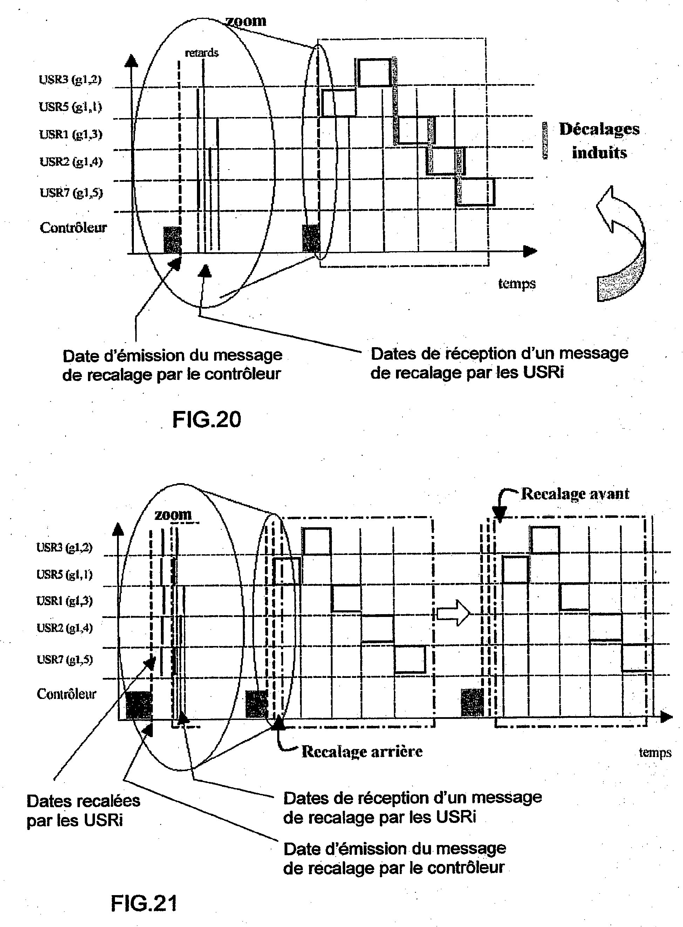

Le temps de propagation n'étant pas nécessairement le même pour atteindre chaque USR, chaque USR est donc associée à un retard constant dans le temps (topologie et distances préservées) qui constitue un risque de chevauchement des intervalles qui induit un risque de collision (accès non déterministe). Ce risque de collision, lié au fait que plusieurs USRs peuvent se retrouver en même temps en possession d'un DDP, est illustré sur la figure 20.

Chaque USR doit donc "recaler" son intervalle temporel afin de minimiser le risque de collision. On peut également limiter le risque au moyen de bornes intérieures dans l'intervalle temporel.

Pour se recaler, chaque USR estime le temps de transmission du message provenant du contrôleur CR. Cette estimation se fait par exemple pendant une phase d'initialisation et à l'invitation du contrôleur CR. Elle consiste à mesurer le temps aller/retour RTT (ou "Round Trip Time") d'un message de longueur donnée entre l'USR et le contrôleur CR. La mesure de RTT/2 est supposée correspondre au temps de propagation aller du message, la topologie étant considérée comme fixe et les distances étant considérées comme préservées lors des mouvements induits par les stimulations.

Le recalage avec la mesure RTT/2 permet aux USRs de mieux se positionner entre elles, cependant ce recalage les positionne incorrectement par rapport à la date de début de la fenêtre, comme illustré sur la figure 21. Par exemple, en calculant ainsi la date de début de la fenêtre temporelle, on effectue un "recalage arrière" qui établit la date de début de l'intervalle de l'USR5 (ici la 1ère de son groupe) avant sa date courante (c'est-à-dire avant qu'elle ne reçoive son attribution). Il est donc avantageux de procéder à un "recalage avant" d'une demi-période D/2. Le contrôleur CR intègre également ce recalage avant dans la détermination de la date de fin de fenêtre temporelle, laquelle correspond à l'instant où il reprend automatiquement la maîtrise du DDP de groupe.

Le positionnement des USRs est donc distribué, du fait que chaque USR se positionne indépendamment.

L'accès au médium, décrit ci-avant, peut être encore optimisé. Plus précisément, il est possible d'optimiser (ou minimiser) le temps de "rotation" d'un DDP individuel au sein d'un groupe grâce à un mécanisme d'anticipation. Pour ce faire, l'USR qui dispose d'un niveau de priorité donné peut utiliser son DDP individuel si au bout d'un temps choisi l'USR disposant du niveau de priorité plus élevé précédent n'a pas émis (ou du moins qu'aucune trame n'a été détectée pendant ledit temps choisi). On définit alors un droit de parole à intervalles glissants (DDPIG).

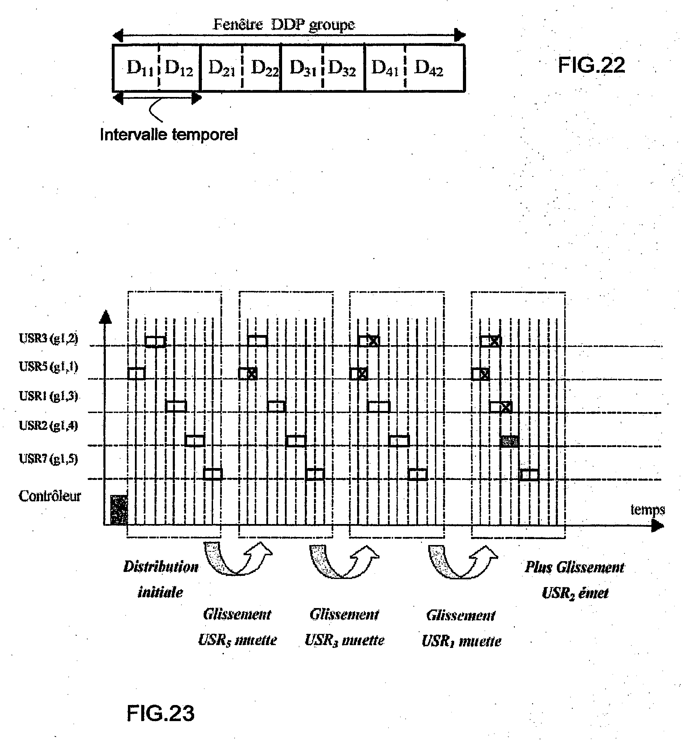

Comme cela est illustré à titre d'exemple non limitatif sur la figure 22, un intervalle temporel peut être subdivisé en deux parties; une première partie Di1 (ici i = 1 à 4), dite de parole, pendant laquelle l'USR peut émettre un message de notification d'erreur, et une seconde partie Di2 réservée à une éventuelle réaction du contrôleur CR. La possibilité de réaction du contrôleur CR, consécutivement à un signalement d'erreur par une USR, est ainsi privilégiée en lui garantissant dans chaque intervalle temporel un temps d'accès au médium pendant lequel les USRs n'ont pas le droit d'émettre. Cependant, cette seconde partie Di2 est uniquement réservée si l'USR en possession du DDP en cours a notifié une erreur.

Comme cela est illustré sur la figure 23, la règle de glissement repose préférentiellement sur l'écoute de la première partie D(i-1)1 de l'intervalle temporel précédent. Si le médium a été occupé, c'est-à-dire si un message a circulé, c'est que l'USR précédente a utilisé son DDP. Par conséquent, la seconde partie D(i-1)2 de l'intervalle temporel précédent est susceptible d'être utilisée en réaction par le contrôleur CR. Dans le cas contraire, c'est-à-dire lorsque la première partie D(i-1)1 de l'intervalle temporel précédent n'a pas été utilisée, chaque USR fait glisser son intervalle temporel sur la seconde partie D(i-1)2 précédente, utilisant ainsi le créneau temporel réservé à la réaction du contrôleur CR. Si aucune USR ne notifie d'erreur pendant toute la durée de la fenêtre temporelle allouée à un groupe, celle-ci peut donc être réduite d'un rapport : (N-1) / (2N), où N est le nombre d'USRs appartenant au groupe concerné.

Plusieurs variantes de gestion des DDPs individuels peuvent être envisagées en présence de notification(s) d'erreur par des USRs.

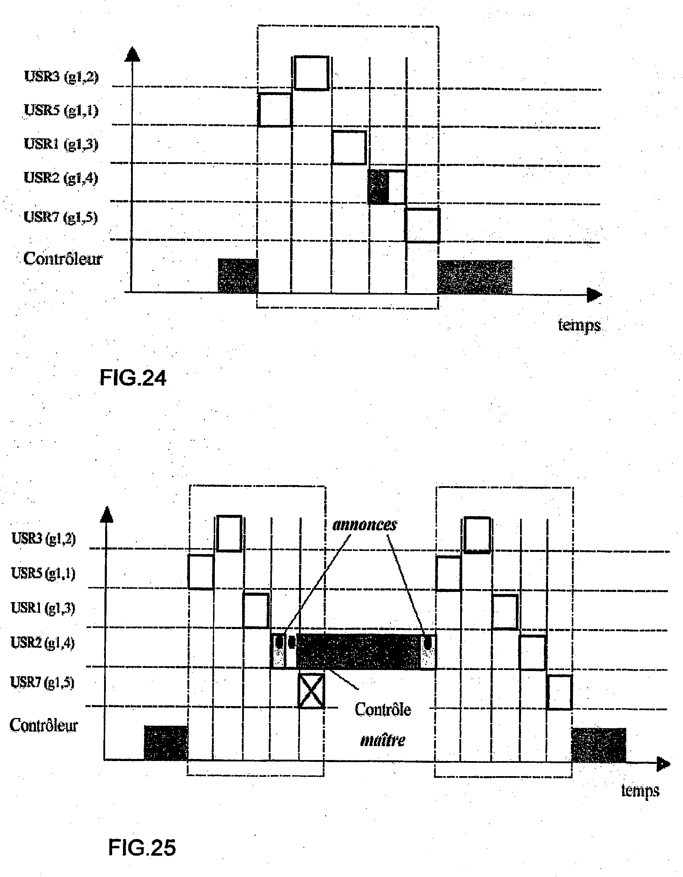

Une première variante, illustrée sur la figure 24, peut consister à permettre à chaque USR d'émettre librement dans son seul intervalle temporel, sachant que les messages d'erreur sont des messages très courts ("Very Short Message"). Le vecteur d'erreur fait en effet 2 octets, ce qui correspond à une trame de 5 octets.

Cette première variante offre une durée d'attribution du DDP fixée et non extensible.

Une deuxième variante peut consister, si l'on suppose que les messages ne sont pas courts, à permettre au maître d'attribuer un intervalle temporel plus long au noeud concerné via un DDP individuel (puis éventuellement restaurer un DDP de groupe). Dans ce cas, d'une part, l'USR concernée se voit octroyer un intervalle temporel plus long et les intervalles temporels des autres USRs sont annulés (mais potentiellement réétablis ultérieurement eh fonction du nouvel octroi de DDP de groupe).

Cette deuxième variante confère un contrôle total du maître sur l'extension de la durée de chaque DDP individuel.

Une troisième variante, illustrée sur la figure 25, peut consister à permettre à chaque USR d'annoncer (ou diffuser) pendant son intervalle temporel sa libre réservation du DDP de groupe, c'est-à-dire le fait qu'elle inhibe le DDP de groupe des autres USRs. Si cette réservation du DDP de groupe est théoriquement sans limite de temps (et donc sans limite de taille), elle reste cependant, préférentiellement, sous le contrôle du maître, afin qu'il puisse intervenir pour la refuser. L'USR, ayant effectuée une libre réservation du DDP du groupe, est également chargée de libérer ensuite ledit DDP de groupe, ce qui induit le repositionnement (ou "reset") des intervalles temporels des autres USRs.

Cette troisième variante offre donc une solution intermédiaire dans laquelle chaque USR est libre d'étendre la durée de son DDP sous le contrôle du maître.

Le partage du médium décrit ci-avant offre un compromis satisfaisant entre déterminisme et réactivité. Il permet de supporter une notification d'erreur de type événementiel de la part des USRs. Cependant, il est possible de procéder différemment, par exemple en permettant des notifications régulières de la part des USRs par échange périodique d'une espèce de descripteur d'état attestant ou non d'un fonctionnement correct. En effet, l'attribution du droit de parole présenté est unique, du fait que les noeuds ne disposent que d'une opportunité pour parler. Mais, une attribution répétitive est également possible. Dans ce cas, les noeuds disposent d'une opportunité "périodique" pour parler, et le contrôleur CR n'a pas à réitérer ses attributions. Il suffit de préciser la taille du groupe et l'USR en déduit la périodicité de son intervalle temporel.

La gestion des transmissions au sein du contrôleur CR et des unités de stimulation réparties (ou USRs) peut être réalisée au moyen de modules de gestion se présentant sous la forme de circuits électroniques, de modules logiciels (ou informatiques), ou d'une combinaison de modules logiciels et de circuits électroniques.

L'invention ne se limite pas aux modes de réalisation de dispositif de répartition de courant (ou étage de sortie), de miroirs de courant multi-sorties reconfigurable, d'électronique de commande, d'unité de stimulation répartie, et d'installation de stimulation décrits ci-avant, seulement à titre d'exemple, mais elle englobe toutes les variantes que pourra envisager l'homme de l'art dans le cadre des revendications ci-après.

**Revendications**

1.  Dispositif (ES) de répartition de courant entre n cathodes (Ki) d'au moins une électrode multipolaire de stimulation (EM) comportant en outre au moins une anode (A), n étant supérieur ou égal à deux, et un miroir de courant reconfigurable (MC) comportant n sorties (K'i) propres à être couplées respectivement auxdites n cathodes (Ki), **caractérisé en ce que** ledit miroir de courant (MC) étant adapté pour recevoir une commande sélectionnant, pour chaque sortie, parmi plusieurs valeurs, une valeur d'un coefficient fixant le rapport entre un courant délivré sur ladite sortie et un courant de commande (Idac) du miroir ; ledit miroir de courant reconfigurable (MC) étant adapté pour délivrer sur lesdites n sorties (K'i) n fractions (Iki) complémentaires dudit courant de commande (Idac), le rapport entre le courant délivré sur chacune des n sorties et le courant de commandé (Idac) du miroir de courant (MC) étant fixé par la valeur du coefficient sélectionnée pour ladite sortie, lesdites valeurs de coefficient sélectionnées pour les sorties définissant une localisation spatiale de la stimulation.

2.  Dispositif selon la revendication 1, **caractérisé en ce que** ledit miroir de courant multi-sorties reconfigurable (MC) est de type dit à répartiteur.

3.  Dispositif selon la revendication 2, **caractérisé en ce que** ledit miroir de courant multi-sorties reconfigurable à répartiteur (MC) comporte un convertisseur courant-tension (CCT) couplé à un convertisseur tension-courant (CTC) et à un répartiteur de courant équilibré contrôlable (RCEC) à m sorties.

4.  Dispositif selon la revendication 3, **caractérisé en ce que** ledit convertisseur courant-tension (CCT) comprend au moins une borne d'entrée (E) propre à absorber un courant (Ie), une borne de masse (M) et une borne de sortie (S) et est agencé pour établir une différence de potentiel choisie (Vsm) entre ladite borne de sortie (S) et ladite borne de masse (M), fonction dudit courant (Ie), **en ce que** ledit convertisseur tension-courant (CTC) comprend au moins une borne d'entrée (E), une borne de masse (M) et une borne de sortie (S). propre à absorber un courant (Is), **en ce que** ledit répartiteur de courant équilibré contrôlable (RCEC) comprend au moins une borne d'entrée (E) propre à délivrer un courant (Ie), un bus de sorties (Sr[1:m]) absorbant chacune un courant (ISi) et un bus de commande (C[1:m]) propre à recevoir des signaux logiques, et **en ce que** ladite borne d'entrée (E) dudit convertisseur courant-tension (CCT) est reliée à une borne d'entrée (E) dudit miroir de courant multi-sorties reconfigurable à répartiteur (MC), ladite borne de masse (M) dudit convertisseur courant-tension (CCT) et lesdites bornes de masse (M) dudit convertisseur tension-courant (CTC) et du répartiteur de courant équilibré contrôlable (RCEC) sont reliées à une borne de masse (M) dudit miroir de courant multi-sorties reconfigurable à répartiteur (MC), ladite borne d'entrée (E) dudit répartiteur de courant équilibré contrôlable (RCEC) est reliée à ladite borne de sortie (S) dudit convertisseur tension-courant CTC, ledit bus de commande (C[1:m]) dudit répartiteur, de courant équilibré contrôlable (RCEC) est relié à un bus de commande (CA[1:p][1:q]) dudit miroir de courant mufti-sorties reconfigurable à répartiteur (MC), et ledit bus de sorties (Sr[1:m]) dudit répartiteur de courant équilibré contrôlable (RCEC) est relié à un bus de sortie (S[1:p]) dudit miroir de courant multi-sorties reconfigurable, à répartiteur, (MC).

5.  Dispositif selon l'une des revendications 3 et 4, caractérise en ce ledit convertisseur courant-tension (CCT) et ledit convertisseur tension-courant (CTC) présentent des architectures appariées.

6.  Dispositif selon la revendication 1, **caractérisé en ce que** ledit miroir de courant multi-sorties reconfigurable (MC) est de type modulaire.

7.  Dispositif selon la revendication 6, **caractérisé en ce que** ledit miroir de courant multi-sorties reconfigurable modulaire (MC) comporte au moins deux convertisseurs tension-courant à transconductance programmable (CTCTP).

8.  Dispositif selon la revendication 6, **caractérisé en ce que** ledit miroir de courant multi-sorties reconfigurable modulaire (MC) comporte un convertisseur courant-tension (CCT) couplé à p convertisseurs tension-courant à transconductance programmable (CTCTP).

9.  Dispositif selon la revendication 8, **caractérise en ce que** ledit convertisseur courant-tension (CCT) comprend au moins une borne d'entrée (E) propre à absorber une courant (Ie), une borne de masse (M) et une borne de sortie (S) et est agencé pour établir une différence de potentiel choisie (Vsm) entre ladite borne de sortie (S) et ladite borne de masse (M), fonction dudit courant (Ie), **en ce que** chaque convertisseur tension-courant à transconductance programmable (CTCTP) comprend au moins une borne d'entrée (E), une borne de masse (M), une borne de sortie (S) propre à absorber un courant (It) et un bus de commande (C[1:p]) propre à recevoir des signaux logiques, et **en ce que** i) ladite borne d'entrée (E) dudit convertisseurs courant-tension (CCT) est reliée à une borne du (E) dudit miroir de courant multi-sorties reconfigurable, modulaire (MC). ii) ladite borne de masse (M) dudit convertisseur courant-tension (CCT) et lesdites bornes de masse (M) desdits p convertisseurs tension-courant à zu programmable (CTCTP) sont reliées à ladite borne de massé M dudit miroir de courant multi-sorties reconfigurable, modulaire (MC), iii) ladite borne d'entrée (E) de chacun desdits p convertisseurs tension-courant à transconductance programmable (CTCTP) est reliée à ladite borne de sortie (S) dudit convertisseur courant-tension (CCT), Iv) ladite borne de sortie (S) de chacun desdits p convertisseurs tension-courant à transconductance programmable (CTCTP) est reliée à l'une desdites sorties (Ki) dudit miroirs de courant multi-sorties reconfigurable, modulaire, (MC), et v) ledit bus de commande C[1:p] de chaque convertisseurs tension-courant à transconductance programmable (CTCTP) est relié à des sous-bus de commande (CA[i][1:p]) dudit miroir de courant multi-sorties reconfigurable modulaire (MC).

10. Dispositif selon l'une des revendications 8 et 9, caractérisé en ce ledit convertisseur courant-tension (CCT) et lesdits p convertisseurs tension-courant à transconductance programmable (CTCTP) présentent des architectures appariées.

11. Dispositif selon rune des revendications 1 à 10, **caractérisé en ce que** le rapport (Ist/Idac) entre le courant (Ist) circulant dans ladite anode (A), égal à la somme des courants (Iki) délivrés sur lesdites sorties (K'I), et ledit courant de commande (Idac) est configurable.

12. Dispositif selon rune des revendications 1 à 10, **caractérisé en ce que** le rapport (Ist/Idac) entre le courant (Ist) circulant dans ladite anode (A), égal à la somme des courants (Iki) délivrés sur lesdites sortes (K'i), et ledit courant de commande (Idac) n'est pas configurable.

13. Dispositif selon rune des revendications 1 à 12, **caractérisé en ce qu'**il comprend un ensemble de n capacités (RCAP) assurant chacune le couplage de l'une desdites sorties (K'i) avec l'une desdites cathodes (Ki).

14. Dispositif selon l'une des revendications 1 à 13, **caractérisé en ce qu'**il comprend un dispositif de surveillance de tension (DST) raccordé auxdites sorties (K'i) et agencé pour mesurer les tensions respectivement présentais au niveau desdites sorties (K'i) de sorte qu'elles permettent un ajustement d'une polarisation de ladite anode (A) de l'électrode multipolaire (EM), via un module d'alimentation haute tension (AHT).

15. Dispositif selon la revendication 14. **caractérise en ce que** ledit dispositif de surveillance de tension (DST) comprend un réseau de convertisseurs analogique-numérique.

16. Dispositif selon la revendication 14 **caractérisé en ce que** ledit dispositif de surveillance de tension (DST) comprend un réseau de n comparateurs de tension agencés chacun pour comparer les n tensions au niveau desdites sorties (K'i) par rapport à une tension de référence commune.

17. Dispositif selon la revendication 14, **caractérisé en ce que** ledit dispositif de surveillance de tension (DST) comprend un réseau de 2n comparateurs de tension agencés par paire pour comparer les n tensions au niveau desdites sorties (K'i) par rapport à deux tensions de référence communes.

18. Dispositif selon l'une des revendications 1 à 17, **caractérisé en ce qu'**il comprend un dispositif de contrôle de décharge (DCD) couplé auxdites sorties (K'i) et à ladite anode (A) et agencé pour établir en fin de stimulation un chemin de conduction entre chacune desdites sorties (K'i) et ladite anode (A), de manière à induire une circulation de n courants de décharge desdites cathodes (Ki) vers ladite anode (A).

19. Dispositif selon la combinaison des revendications 13 et 18. caractérisé en ce lesdits n courants de décharge proviennent des n énergies respectivement accumulées par lesdites n capacités dudit ensemble (RCAP).

20. Dispositif selon l'une des revendications 18 et 19, **caractérisé en ce que** ledit dispositif de contrôle de décharge (DCD) est agencé pour limiter chaque courant de décharge a une fraction de la valeur maximale du courant de stimulation dégivré sur la sortie (K'i) associée.

**21.** Electronique de commande (EC) **caractérisée en ce qu'**elle comprend i) au moins un dispositif (ES) selon l'une des revendications précédentes. ii) un convertisseur numérique-analogique (DAC) propre à convertir une consigne d'amplitude de courant (Csgn) en un courant analogique de commande (idac) et couplé audit dispositif (ES), pour lui fournir ledit courant de commande (idac), et iii) un module d'alimentation haute tension (AHT) couplé au moins à ladite anode (A) et agencé pour polariser ladite anode (A) sous une tension choisie de sorte qu'elle permette la circulation des courants imposés à chaque cathode (Ki) par ledit dispositif (ES).

**22.** Electronique de commande selon la revendication 21, **caractérisée en ce que** ledit convertisseur numérique-analogique (DAC) présente une architectures dite « à source de courant unitaire » propre à garantir la monotonicité de sa fonction de conversion.

**23.** électronique de commande selon l'une des revendications 21 et 22, **caractérisée en ce que** ledit module d'alimentation haute tension (AHT) est un convertisseur de type « continu-continu ».

**24.** Electronique de commande selon la revendication 23, **caractérisée en ce que** ledit module d'alimentation haute tension (AHT) est un hacheur à stockage inductif.

**25.** Electronique de commande selon la revendication 23, **caractérisée en ce que** ledit module d'alimentation haute tension (AHT) comporte une pompe de charges à stockage capacitif.

**26.** Electronique de commande selon la revendication 25, **caractérisée en ce que** ledit module d'alimentation haute tension (AHT) comporte en outre un multiplexeur de tension (MUX) couplé à ladite pompe de charges.

**27.** Electronique de commande selon la revendication 26, **caractérisée en ce que** ledit module d'alimentation haute tension (AHT) est agencé pour fonctionner en régime continu ou en régime discontinu.

**28.** Unité de stimulation répartie (I, USR) comportant au moins une électrode multipolaire (EM) comprenant au moins une anode (A) et au moins deux cathodes (Ki), **caractérisée en ce qu'**elle comprend en outre au moins une électronique de commande (EC) selon l'une des revendications 21 à 27.

**29.** Unité de stimulation répartie selon la revendication 28, **caractérisée en ce qu'**elle comprend un contrôleur numérique (CN) propre à délivrer ladite consigne d'amplitude de courant (Csgn) et à définir les valeurs desdites fractions de courant (Iki) délivrées sur lesdites sorties (K'i) dudit miroir de courant multi-sorties reconfigurable (MC).

**30.** Unité de stimulation répartie selon la revendication 29, **caractérisée en ce que** ledit contrôleur numérique (CN) et ladite électronique de commande (EC) constituent respectivement une partie numérique et une partie analogique d'un ASIC de type mixte.

**31.** Unité de stimulation répartie selon l'une des revendications 28 à 30, **caractérisée en ce qu'**elle comprend des moyens de transmission par voie d'ondes (MT) et des moyens de gestion agencés pour gérer ladite transmission de données selon un protocole choisi entre elle et un contrôleur (CR) d'une installation de stimulation (IS).

**32.** Unité de stimulation répartie selon l'une des revendications 28 à 30, **caractérisée en ce qu'**elle comprend des moyens de transmission par bus filaire (MT) et des moyens de gestion agencés pour gérer la transmissions de données selon un protocole choisi entre elle et un contrôleur (CR) d'une installation de stimulation (IS).

**33.** Unité de stimulation répartie selon l'une des revendications 28 à 32, **caractérisée en ce que** ledit contrôleur numérique (CN) est agencé pour déduire, à partir des valeurs des courants de stimulation imposés, de la tension de sortie dudit module d'alimentation haute tension (AHT) et des mesures de tension effectuées par ledit dispositif de surveillance de tension (DST) aux bornes des sorties (K'i) dudit miroir de courant multi-sorties reconfigurable (MC), l'impédance (Zi) de chaque electrode (Ki), de manière à contrôler ladite polarisation de l'anode (A).

**34.** Unité de stimulation répartie selon l'une des revendications 28 à 33, **caractérisée en ce qu'**elle constitue un implant (I).

**35.** installation de stimulation (IS), **caractérisée en ce qu'**elle comprend au moins une unité de stimulation répartie (I, USR) selon l'une des revendications 28 à 34, et un contrôleur (CR) agencé pour échanger des données avec chaque unité de stimulation répartie (I. USR).

**36.** Installation de stimulation selon la revendication 35, dans laquelle le contrôleur de l'installation (CR) et l'au moins une unité de stimulation répartie (I, USR) sont adaptés pour communiquer via un medium, l'accès audit médium étant géré selon un principe de droit de parole de groupe(s) d'unités de stimulation répartie (I, USR) à intervalles glissants, basé sur un positionnement automatique d'intervalles temporels dépendant de niveaux de priorité associés respectivement à chaque noeud au sein de son groupe et de caractéristiques topologiques.

**37.** Installation de stimulation selon la revendication 36, **caractérisé en ce que** lesdites caractéristiques topologiques comprennent au moins un débit de données et un temps de propagation.

**38.** Installation de stimulation selon l'une des revendications 36 et 37, **caractérisé en ce que** ladite gestion d'accès au médium est agencée pour optimiser l'exploitation de la bande passante.

**39.** Installation de stimulation (IS) selon l'une des revendications 35 à 38, pour la stimulation de nerf(s) et/ou de muscle(s) d'animal ou d'humain.

**Patentansprüche**

**1.** Vorrichtung (ES) zur Stromverteilung zwischen n Kathoden (Ki) mindestens einer mehrpoligen Stimulationselektrode (EM), darüber hinaus mindestens eine Anode (A), wobei n größer als oder gleich Zwei ist, und einen umkonfigurierbaren Stromspiegel (MC), n Ausgänge (K'i) umfassend, die sich dazu eignen, jeweils an die n Kathoden (Ki) angeschlossen zu werden, **dadurch gekennzeichnet, dass** der Stromspiegel (MC) dazu angepasst ist, einen Befehl zu empfangen, der für jeden Ausgang aus mehreren Werten einen Wert eines Koeffizienten auswählt, der das Verhältnis zwischen einem am Ausgang ausgegebenen Strom und einem Steuerstrom (Idac) des Spiegels festlegt; wobei der umkonfigurierbare Stromspiegel (MC) dazu angepasst ist, an den n Ausgängen (Ki'n) n komplementäre Bruchteile (Iki) des Steuerstroms (Idac) auszugeben, wobei das Verhältnis zwischen dem an jedem der n Ausgänge ausgegebenen Strom und dem Steuerstrom (Idac) des Stromspiegels (MC) durch den für den Ausgang ausgewählten Wert des Koeffizienten festgelegt wird, wobei die für die Ausgänge ausgewählten Koeffizientenwerte eine räumliche Lokalisierung der Stimulation definieren.

**2.** Vorrichtung nach Anspruch 1, **dadurch gekennzeichnet, dass** der umkonfigurierbare Mehrfachausgangs-Stromspiegel (MC) vom sogenannten Typ mit Verteiler ist.

**3.** Vorrichtung nach Anspruch 2, **dadurch gekennzeichnet, dass** der umkonfigurierbare Mehrfachausgangs-Stromspiegel mit Verteiler (MC) einen Strom-/Spannungswandler (CCT) umfasst, der an einen Spannungs-/Stromwandler (CTC) und einen steuerbaren symmetrischen Stromverteiler (RCEC) mit m Ausgängen angeschlossen ist.

**4.** Vorrichtung nach Anspruch 3, **dadurch gekennzeichnet, dass** der Strom-/Spannungswandler (CCT) mindestens einen Eingangsanschluss (E), der sich dazu eignet, einen Strom (Ie) aufzunehmen, einen Masseanschluss (M) und einen Ausgangsanschluss (S) umfasst und dazu eingerichtet ist, einen ausgewählten Potentialunterschied (Vsm) zwischen dem Ausgangsanschluss (S) und dem Masseanschluss (M) in Abhängigkeit vom Strom (Ie) herzustellen, dass der Spannungs-/Stromwandler (CTC) mindestens einen Eingangsanschluss (E), einen Masseanschluss (M) und einen Ausgangsanschluss (S) umfasst, der sich dazu eignet, einen Strom (Is) aufzunehmen, dass der steuerbare symmetrische Stromverteiler (RCEC) mindestens einen Eingangsanschluss (E), der sich dazu eignet, einen Strom (Ie) auszugeben, einen Bus von Ausgängen (Sr[1:m]), die jeweils einen Strom (Isi) aufnehmen, und einen Steuerbus (C[1:m]) umfasst, der sich dazu eignet, logische Signale zu empfangen, und dass der Eingangsanschluss (E) des Strom-/Spannungswandlers (CCT) mit einem Eingangsanschluss (E) des umkonfigurierbaren Mehrfachausgangs-Stromspiegels mit Verteiler (MC) verbunden ist, der Masseanschluss (M) des Strom-/Spannungs-wandlers (CCT) und die Masseanschlüsse (M) des Spannungs-/Stromwandlers (CTC) und des steuerbaren symmetrischen Stromverteilers (RCEC) mit einem Masseanschluss (M) des umkonfigurierbaren Mehrfachausgangs-Stromspiegels mit Verteiler (MC) verbunden sind, der Eingangsanschluss (E) des steuerbaren symmetrischen Stromverteilers (RCEC) mit dem Ausgangsanschluss (S) des Spannungs-/Stromwandlers (CTC) verbunden ist, der Steuerbus (C[1:m]) des steuerbaren symmetrischen Stromverteilers (RCEC) mit einem Steuerbus (CA[1:p][1:q]) des umkonfigurierbaren Mehrfachausgangs-Stromspiegels mit Verteiler (MC) verbunden ist, und der Bus von Ausgängen (Sr[1:m]) des steuerbaren symmetrischen Stromverteilers (RCEC) mit einem Ausgangsbus (S[1:P]) des umkonfigurierbaren Mehrfachausgangs-Stromspiegels mit Verteiler (MC) verbunden ist.

**5.** Vorrichtung nach einem der Ansprüche 3 und 4, **dadurch gekennzeichnet, dass** der Strom-/Spannungswandler (CCT) und der Spannungs-/Stromwandler (CTC) gepaarte Architekturen aufweisen.

6. Vorrichtung nach Anspruch 1, **dadurch gekennzeichnet, dass** der umkonfigurierbare Mehrfachausgangs-Stromspiegel (MC) vom modularen Typ ist.

7. Vorrichtung nach Anspruch 6, **dadurch gekennzeichnet, dass** der umkonfigurierbare Mehrfachausgangs-Stromspiegel (MC) mindestens zwei Spannungs-/Stromwandler mit programmierbarer Transduktanz (CTCTP) umfasst.

8. Vorrichtung nach Anspruch 6, **dadurch gekennzeichnet, dass** der modulare umkonfigurierbare Mehrfachausgangs-Stromspiegel (MC) einen Strom-/Spannungswandler (CCT) umfasst, der an p Spannungs-/Stromwandler mit programmierbarer Transduktanz (CTCTP) angeschlossen ist.

9. Vorrichtung nach Anspruch 8, **dadurch gekennzeichnet, dass** der Strom-/Spannungswandler (CCT) mindestens einen Eingangsanschluss (E), der sich dazu eignet, einen Strom (Ie) aufzunehmen, einen Masseanschluss (M) und einen Ausgangsanschluss (S) umfasst und dazu eingerichtet ist, einen ausgewählten Potentialunterschied (Vsm) zwischen dem Ausgangsanschluss (S) und dem Masseanschluss (M) in Abhängigkeit vom Strom (Ie) herzustellen, dass der Spannungs-/Stromwandler mit programmierbarer Transduktanz (CTCTP) mindestens einen Eingangsanschluss (E), einen Masseanschluss (M) und einen Ausgangsanschluss (S), der sich dazu eignet, einen Strom (It) aufzunehmen, und einen Steuerbus (C[1:p]) umfasst, der sich dazu eignet, logische Signale zu empfangen, und dass i) der Eingangsanschluss (E) des Strom-/Spannungswandlers (CCT) mit einem Eingangsanschluss (E) des modularen umkonfigurierbaren Mehrfachausgangs-Stromspiegels (MC) verbunden ist, ii) der Masseanschluss (M) der p Spannungs-/Stromwandler mit programmierbarer Transduktanz (CTCTP) mit dem Masseanschluss (M) des modularen umkonfigurierbaren Mehrfachausgangs-Stromspiegels (MC) verbunden ist, iii) der Eingangsanschluss (E) jedes der p Spannungs-/Stromwandler mit programmierbarer Transduktanz (CTCTP) mit dem Ausgangsanschluss (S) des Strom-/Spannungswandlers (CCT) verbunden ist, iv) der Ausgangsanschluss (S) jedes der p Spannungs-/Stromwandler mit programmierbarer Transduktanz (CTCTP) mit einem der Ausgänge (Ki) des modularen umkonfigurierbaren Mehrfachausgangs-Stromspiegels (MC) verbunden ist, und v) der Steuerbus C[1 :p] jedes Spannungs-/Stromwandlers mit programmierbarer Transduktanz (CTCTP) mit Steuerunterbussen (CA[i][1 :p]) des modularen umkonfigurierbaren Mehrfachausgangs-Stromspiegels (MC) verbunden ist.

10. Vorrichtung nach einem der Ansprüche 8 und 9, **dadurch gekennzeichnet, dass** der Strom-/Spannungswandler (CCT) und die p Spannungs-/Stromwandler mit programmierbarer Transduktanz (CTCTP) gepaarte Architekturen aufweisen.

11. Vorrichtung nach einem der Ansprüche 1 bis 10, **dadurch gekennzeichnet, dass** das Verhältnis (Ist/Idac) zwischen dem in der Anode (A) fließenden Strom (Ist) gleich der Summe der an den Ausgängen (K'I) ausgegebenen Ströme (Iki) und der Steuerstrom (Idac) konfigurierbar ist.

12. Vorrichtung nach einem der Ansprüche 1 bis 10, **dadurch gekennzeichnet, dass** das Verhältnis (Ist/Idac) zwischen dem in der Anode (A) fließenden Strom (Ist) gleich der Summe der an den Ausgängen (K'I) ausgegebenen Ströme (Iki) und der Steuerstrom (Idac) nicht konfigurierbar ist.

13. Vorrichtung nach einem der Ansprüche 1 bis 12, **dadurch gekennzeichnet, dass** sie eine Gruppe von n Kapazitäten (RCAP) umfasst, die jeweils die Zusammenschaltung eines der Ausgänge (K'i) mit einer der Kathoden (KI) sicherstellen.

14. Vorrichtung nach einem der Ansprüche 1 bis 13, **dadurch gekennzeichnet, dass** sie eine Spannungsüberwachungsvorrichtung (DST) umfasst, die an die Ausgänge (K'i) angeschlossen und dazu eingerichtet ist, die jeweils an den Ausgängen (K'i) anliegenden Spannungen zu messen, so dass diese eine Anpassung einer Polarisation der Anode (A) der mehrpoligen Elektrode (EM) über ein Hochspannungsspeisemodul (AHT) zulassen.

15. Vorrichtung nach Anspruch 14, **dadurch gekennzeichnet, dass** die Spannungsüberwachungsvorrichtung (DST) ein Netzwerk von Analog/Digital-Wandlern umfasst.

16. Vorrichtung nach Anspruch 14, **dadurch gekennzeichnet, dass** die Spannungsüberwachungsvorrichtung (DST) ein Netzwerk von n Spannungskomparatoren umfasst, die jeweils dazu eingerichtet sind, die n Spannungen an den Ausgängen (K'i) gegenüber einer gemeinsamen Referenzspannung zu vergleichen.

17. Vorrichtung nach Anspruch 14, **dadurch gekennzeichnet, dass** die Spannungsüberwachungsvorrichtung (DST) ein Netzwerk von 2n Spannungskomparatoren umfasst, die paarweise dazu eingerichtet sind, die n Spannungen an den Ausgängen (K'i) gegenüber zwei gemeinsamen Referenzspannungen zu vergleichen.

18. Vorrichtung nach einem der Ansprüche 1 bis 17, **dadurch gekennzeichnet, dass** sie eine Entladekontrollvorrichtung (DCD) umfasst, die an die Ausgänge (K'i) und die Anode (A) angeschlossen und dazu eingerichtet ist, am Stimulationsende eine Leitungsbahn zwischen jeweils den Ausgängen (K'i) und der Anode (A) herzustellen, um einen Fluss von n Entladströmen von den Kathoden (KI) zur Anode (A) zu induzieren.

19. Vorrichtung nach der Kombination der Ansprüche 13 und 18, **dadurch gekennzeichnet, dass** die n Entladeströme von n Energien stammen, die jeweils durch die n Kapazitäten der Gruppe (RCAP) akkumuliert wurden.

20. Vorrichtung nach einem der Ansprüche 18 und 19, **dadurch gekennzeichnet, dass** die Entladekontrollvorrichtung (DCD) dazu eingerichtet ist, jeden Entladestrom auf einen Bruchteil des maximalen Werts des am zugeordneten Ausgang (K'i) ausgegebenen Stimulationsstroms zu begrenzen.

21. Steuerelektronik (EC), **dadurch gekennzeichnet, dass** sie umfasst i) mindestens eine Vorrichtung (ES) nach einem der vorhergehenden Ansprüche, ii) einen Digital/Analog-Wandler (DAC), der sich dazu eignet, einen Stromamplitudeneinstellwert (Csgn) in einen analogen Steuerstrom (Idac) umzusetzen, und an die Vorrichtung (ES) angeschlossen ist, um ihr den Steuerstrom (Idac) bereitzustellen, und iii) ein Hochspannungsspeisemodul (AHT), das zumindest an die Anode (A) angeschlossen und dazu eingerichtet ist, die Anode (A) unter einer Spannung so zu polarisieren, dass sie den Fluss der jeder Kathode (Ki) durch die Vorrichtung (ES) auferlegten Ströme zulässt.

22. Steuerelektronik nach Anspruch 21, **dadurch gekennzeichnet, dass** der Digital/Analog-Wandler (DAC) eine sogenannte "Einheitsstromquellen"-Architektur aufweist, die sich dazu eignet, die Gleichförmigkeit seiner Umsetzungsfunktion zu gewährleisten.

23. Steuerelektronik nach einem der Ansprüche 21 und 22, **dadurch gekennzeichnet, dass** das Hochspannungsspeisemodul (AHT) ein Wandler des Typs "Gleichspannung/Gleichspannung" ist.

24. Steuerelektronik nach Anspruch 23, **dadurch gekennzeichnet, dass** das Hochspannungsspeisemodul (AHT) ein Zerhacker mit induktiver Speicherung ist.

25. Steuerelektronik nach Anspruch 23, **dadurch gekennzeichnet, dass** das Hochspannungsspeisemodul (AHT) eine Ladepumpe mit kapazitiver Speicherung umfasst.

26. Steuerelektronik nach Anspruch 25, **dadurch gekennzeichnet, dass** das Hochspannungsspeisemodul (AHT) darüber hinaus einen Spannungsmultiplexer (MUX) umfasst, der an die Ladepumpe angeschlossen ist.

27. Steuerelektronik nach Anspruch 26, **dadurch gekennzeichnet, dass** das Hochspannungsspeisemodul (AHT) dazu eingerichtet ist, in einem kontinuierlichen oder diskontinuierlichen Betrieb zu arbeiten.

28. Einheit zur verteilten Stimulation (I, USR), mindestens eine mehrpolige Elektrode (EM) mit mindestens einer Anode (A) und mindestens zwei Kathoden (Ki) umfassend, **dadurch gekennzeichnet, dass** sie darüber hinaus mindestens eine Steuerelektronik (EC) nach einem der Ansprüche 21 bis 27 umfasst.

29. Einheit zur verteilten Stimulation nach Anspruch 28, **dadurch gekennzeichnet, dass** sie eine numerische Steuerung (CN) umfasst, die sich dazu eignet, den Stromamplitudeneinstellwert (Csgn) auszugeben und die Werte der Strombruchteile (Iki) zu definieren, die an den Ausgängen (K'i) des umkonfigurierbaren Mehrfachausgangs-Stromspiegels (MC) ausgegeben werden.

30. Einheit zur verteilten Stimulation nach Anspruch 29, **dadurch gekennzeichnet, dass** die numerische Steuerung (CN) und die Steuerelektronik (EC) jeweils einen digitalen Teil und einen analogen Teil eines ASIC des Mischtyps bilden.

31. Einheit zur verteilten Stimulation nach einem der Ansprüche 28 bis 30, **dadurch gekennzeichnet, dass** sie Einrichtungen zur Übertragung über einen Funkweg (MT) und Verwaltungseinrichtungen umfasst, die dazu eingerichtet sind, die Übertragung von Daten gemäß einem Protokoll zu verwalten, das zwischen ihr und einer Kontrollinstanz (CR) einer Stimulationsinstallation (IS) ausgewählt wird.

**32.** Einheit zur verteilten Stimulation nach einem der Ansprüche 28 bis 30, **dadurch gekennzeichnet, dass** sie Einrichtungen zur Übertragung über einen drahtgebundenen Bus (MT) und Verwaltungseinrichtungen umfasst, die dazu eingerichtet sind, die Übertragung von Daten gemäß einem Protokoll zu verwalten, das zwischen ihr und einer Kontrollinstanz (CR) einer Stimulationsinstallation (IS) ausgewählt wird.

**33.** Einheit zur verteilten Stimulation nach einem der Ansprüche 28 bis 32, **dadurch gekennzeichnet, dass** die numerische Steuerung (CN) dazu eingerichtet ist, von den Werten der auferlegten Stimulationsströme, der Ausgangsspannung des Hochspannungsspeisemoduls (AHT) und den Spannungsmessungen, die durch die Spannungsüberwachungsvorrichtung (DST) an den Ausgangsanschlüssen (K'i) des umkonfigurierbaren Mehrfachausgangs-Stromspiegels (MC) durchgeführt wurden, die Impedanz (Zi) jeder Elektrode (Ki) abzuziehen, um die Polarisation der Anode (A) zu überprüfen.

**34.** Einheit zur verteilten Stimulation nach einem der Ansprüche 28 bis 33, **dadurch gekennzeichnet, dass** sie ein Implantat darstellt.

**35.** Stimulationsinstallation (IS), **dadurch gekennzeichnet, dass** sie eine Einheit zur verteilten Stimulation (I, USR) nach einem der Ansprüche 28 bis 34 und eine Steuerung (CR) umfasst, die dazu eingerichtet ist, Daten mit jeder Einheit zur verteilten Stimulation (I, USR) auszutauschen.

**36.** Stimulationsinstallation nach Anspruch 35, wobei die Steuerung der Installation (CR) und die mindestens eine Einheit zur verteilten Stimulation (I, USR) dazu angepasst sind, über ein Medium zu kommunizieren, wobei der Zugang zu dem Medium nach einem Prinzip des Rederechts einer Gruppe/von Gruppen von Einheiten zur verteilten Stimulation (I, USR) in gleitenden Abständen basierend auf einer automatischen Anordnung zeitlicher Abstände in Abhängigkeit von Prioritätsgraden, die jeweils jedem Knoten innerhalb seiner Gruppe zugeordnet sind, und von topologischen Merkmalen verwaltet wird.

**37.** Stimulationsinstallation nach Anspruch 36, **dadurch gekennzeichnet, dass** die topologischen Merkmale mindestens einen Datendurchsatz und eine Ausbreitungszeit umfassen.

**38.** Stimulationsinstallation nach einem der Ansprüche 36 und 37, **dadurch gekennzeichnet, dass** die Verwaltung eines Zugangs zum Medium dazu eingerichtet ist, die Bandbreitennutzung zu optimieren.

**39.** Stimulationsinstallation (IS) nach einem der Ansprüche 35 bis 38 für die Nerven- und/oder Muskelstimulation eines Tiers oder Menschs.

**Claims**

**1.** Device (ES) for distributing power between n cathodes (Ki) of at least one multipolar stimulating electrode (EM) further comprising at least one anode (A), n being equal to or greater than two, and a reconfigurable current mirror (MC) comprising n outputs (K'i) capable of being coupled respectively to said n cathodes (Ki), **characterised in that** said current mirror (MC) is adapted to receive a control selecting, for each output, among several values, a coefficient value fixing the ratio between a current supplied to said output and a control current (Idac) of the mirror; said reconfigurable current mirror (MC) being adapted to supply to said n outputs (K'i) n complementary fractions (Iki) of said control current (Idac), the ratio between the current supplied to each of said n outputs and the control current (Idac) of the current mirror (MC) being fixed by the coefficient value selected for said output, said coefficient values selected for the outputs defining a spatial locating of the stimulation.

**2.** Device according to claim 1, **characterised in that** said reconfigurable multi-output current mirror (MC) is of what is known as the "distributor" type.

**3.** Device according to claim 2, **characterised in that** said distributor-type reconfigurable multi-output current mirror (MC) comprises a current/voltage converter (CCT) coupled to a voltage/current converter (CTC) and to a controllable balanced current distributor (RCEC) having m outputs.

**4.** Device according to claim 3, **characterised in that** said current/voltage converter (CCT) comprises at least one input terminal (E) capable of absorbing a current (Ie), an earth terminal (M) and an output terminal (S) and is configured to establish a chosen potential difference (Vsm) between said output terminal (S) and said earth terminal (M), as a function of said current (Ie), **in that** said voltage/current converter (CTC) comprises at least one input terminal (E), an earth terminal (M) and an output terminal (S) capable of absorbing a current (Is), **in that** said controllable balanced current distributor (RCEC) comprises at least one input terminal (E) capable of supplying a current (Ie), a bus of outputs (Sr[1:m]), each absorbing a current (ISi), and a control bus (C[1:m]) capable of receiving logic signals, and **in that** said input terminal (E) of said current/voltage converter (CCT) is connected to an input terminal (E) of said distributor-type reconfigurable multi-output current mirror (MC), said earth terminal (M) of said current/voltage converter (CCT) and said earth terminals (M) of said voltage/current converter (CTC) and of the controllable balanced current distributor (RCEC) are connected to an earth terminal (M) of said distributor-type reconfigurable multi-output current mirror (MC), said input terminal (E) of said controllable balanced current distributor (RCEC) is connected to said output terminal (S) of said voltage/current converter (CTC), said control bus (C[1:m]) of said controllable balanced current distributor (RCEC) is connected to a control bus (CA[1:p][1:q]) of said distributor-type reconfigurable multi-output current mirror (MC), and said output bus (Sr[1:m]) of said controllable balanced current distributor (RCEC) is connected to an output bus (S[1:p]) of said distributor-type reconfigurable multi-output current mirror (MC).

5. Device according to either claim 3 or claim 4, **characterised in that** said current/voltage converter (CCT) and said voltage/current converter (CTC) have matched architectures.

6. Device according to claim 1, **characterised in that** said reconfigurable multi-output current mirror (MC) is of the modular type.

7. Device according to claim 6, **characterised in that** said modular reconfigurable multi-output current mirror (MC) comprises at least two voltage/current converters with programmable transconductance (CTCTP).

8. Device according to claim 6, **characterised in that** said modular reconfigurable multi-output current mirror (MC) comprises a current/voltage converter (CCT) coupled to p voltage/current converters with programmable transconductance (CTCTP).

9. Device according to claim 8, **characterised in that** said current/voltage converter (CCT) comprises at least one input terminal (E) capable of absorbing a current (Ie), an earth terminal (M) and an output terminal (S) and is configured to establish a chosen potential difference (Vsm) between said output terminal (S) and said earth terminal (M), as a function of said current (Ie), **in that** each voltage/current converter with programmable transconductance (CTCTP) comprises at least one input terminal (E), an earth terminal (M), an output terminal (S) capable of absorbing a current (It) and a control bus (C[1:p]) capable of receiving logic signals, and **in that** i) said input terminal (E) of said current/voltage converter (CCT) is connected to an input terminal (E) of said modular reconfigurable multi-output current mirror (MC), ii) said earth terminal (M) of said current/voltage converter (CCT) and said earth terminals (M) of said p voltage/current converters with programmable transconductance (CTCTP) are connected to said earth terminal M of said modular reconfigurable multi-output current mirror (MC), iii) said input terminal (E) of each of said p voltage/current converters with programmable transconductance (CTCTP) is connected to said output terminal (S) of said current/voltage converter (CCT), iv) said output terminal (S) of each of said p voltage/current converters with programmable transconductance (CTCTP) is connected to one of said outputs (Ki) of said modular reconfigurable multi-output current mirror (MC), and v) said control bus (C[1:p]) of each voltage/current converter with programmable transconductance (CTCTP) is connected to control sub-buses (CA[i][1:p]) of said modular reconfigurable multi-output current mirror (MC).

10. Device according to either claim 8 or claim 9, **characterised in that** said current/voltage converter (CCT) and said p voltage/current converters with programmable transconductance (CTCTP) have matched architectures.

11. Device according to any one of claims 1 to 10, **characterised in that** the ratio (Ist/Idac) between the current (1st) circulating in said anode (A), which is equal to the sum of the currents (Iki) supplied to said outputs (K'i), and said control current (Idac) is configurable.

12. Device according to any one of claims 1 to 10, **characterised in that** the ratio (Ist/Idac) between the current (1st) circulating in said anode (A), which is equal to the sum of the currents (Iki) supplied to said outputs (K'i), and said control current (Idac) is not configurable.

EP 1 789 131 B1

**13.** Device according to any one of claims 1 to 12, **characterised in that** it comprises a set of n capacitors (RCAP), each coupling one of said outputs (K'i) to one of said cathodes (Ki).

**14.** Device according to any one of claims 1 to 13, **characterised in that** it comprises a voltage monitoring device (DST) connected to said outputs (K'i) and configured to measure the voltages respectively present at said outputs (K'i), so they allow adjustment of a polarisation of said anode (A) of the multipolar electrode (EM) via a high-voltage supply module (AHT).

**15.** Device according to claim 14, **characterised in that** said voltage monitoring device (DST) comprises a network of analogue/digital converters.

**16.** Device according to claim 14, **characterised in that** said voltage monitoring device (DST) comprises a network of n voltage comparators, each configured to compare the n voltages at said outputs (K'i) relative to a common reference voltage.

**17.** Device according to claim 14, **characterised in that** said voltage monitoring device (DST) comprises a network of 2n voltage comparators configured in pairs to compare the n voltages at said outputs (K'i) relative to two common reference voltages.

**18.** Device according to any one of claims 1 to 17, **characterised in that** it comprises a discharge control device (DCD) coupled to said outputs (K'i) and to said anode (A) and configured to establish, at the end of the stimulation, a conduction path between each of said outputs (K'i) and said anode (A), so as to induce a circulation of n discharge currents from said cathodes (Ki) to said anode (A).

**19.** Device according to claims 13 and 18 combined, **characterised in that** said n discharge currents originate from the n energies respectively stored by said n capacitors of said set (RCAP).

**20.** Device according to either claim 18 or claim 19, **characterised in that** said discharge control device (DCD) is configured to limit each discharge current to a fraction of the maximum value of the stimulation current supplied to the associated output (K'i).

**21.** Control electronics (EC), **characterised in that** they comprise i) at least one device (ES) according to any one of the preceding claims, ii) a digital/analogue converter (DAC) capable of converting a current amplitude reference value (Csgn) into a control analogue current (Idac) and coupled to said device (ES) to provide it with said control current (Idac), and iii) a high-voltage supply module (AHT) coupled at least to said anode (A) and configured to polarise said anode (A) under a chosen voltage, so it allows circulation of the currents imposed on each cathode (Ki) via said device (ES).

**22.** Control electronics according to claim 21, **characterised in that** said digital/analogue converter (DAC) has what is known as a "unit current source" architecture capable of ensuring the monotonicity of its conversion function.

**23.** Control electronics according to either claim 21 or claim 22, **characterised in that** said high-voltage supply module (AHT) is a "DC/DC"-type converter.

**24.** Control electronics according to claim 23, **characterised in that** said high-voltage supply module (AHT) is an inductive storage chopper.

**25.** Control electronics according to claim 23, **characterised in that** said high-voltage supply module (AHT) comprises a capacitive storage charge pump.

**26.** Control electronics according to claim 25, **characterised in that** said high-voltage supply module (AHT) further comprises a voltage multiplexer (MUX) coupled to said charge pump.

**27.** Control electronics according to claim 26, **characterised in that** said high-voltage supply module (AHT) is configured to operate continuously or discontinuously.

**28.** Distributed stimulation unit (I, USR) comprising at least one multipolar electrode (EM) comprising at least one anode (A) and at least two cathodes (Ki), **characterised in that** it further comprises at least one control electronics (EC) according to any one of claims 21 to 27.

29. Distributed stimulation unit according to claim 28, **characterised in that** it comprises a digital controller (CN) capable of supplying said current amplitude reference value (Csgn) and of defining the values of said current fractions (Iki) supplied to said outputs (K'i) of said reconfigurable multi-output current mirror (MC).

30. Distributed stimulation unit according to claim 29, **characterised in that** said digital controller (CN) and said control electronics (EC) respectively form a digital part and an analogue part of a mixed-type ASIC.

31. Distributed stimulation unit according to any one of claims 28 to 30, **characterised in that** it comprises wave transmission means (MT) and management means configured to manage said data transmission in accordance with a protocol chosen between it and a controller (CR) of a stimulation system (IS).

32. Distributed stimulation unit according to any one of claims 28 to 30, **characterised in that** it comprises wired bus transmission means (MT) and management means configured to manage the data transmission in accordance with a protocol chosen between it and a controller (CR) of a stimulation system (IS).

33. Distributed stimulation unit according to any one of claims 28 to 32, **characterised in that** said digital controller (CN) is configured to deduce, from the values of the imposed stimulation currents, from the output voltage of said high-voltage supply module (AHT) and from the voltage measurements carried out by said voltage monitoring device (DST) at the terminals of the outputs (K'i) of said reconfigurable multi-output current mirror (MC), the impedance (Zi) of each electrode (Ki), so as to control said polarisation of the anode (A).

34. Distributed stimulation unit according to any one of claims 28 to 33, **characterised in that** it forms an implant (I).

35. Stimulation system (IS), **characterised in that** it comprises at least one distributed stimulation unit (I, USR) according to any one of claims 28 to 34 and a controller (CR) configured to exchange data with each distributed stimulation unit (I, USR).

36. Stimulation system according to claim 35, wherein the controller of the system (CR) and said at least one distributed stimulation unit (I, USR) are adapted to communicate via a medium, the access to said medium being managed in accordance with a principle of the right to speak of group(s) of distributed stimulation units (I, USR) at sliding intervals, based on an automatic positioning of time intervals which is dependent on levels of priority respectively associated with each node within its group and on topological characteristics.

37. Stimulation system according to claim 36, **characterised in that** said topological characteristics comprise at least a data rate and a propagation time.

38. Stimulation system according to either claim 36 or claim 37, **characterised in that** said means for managing access to the medium is configured to optimise the exploitation of the bandwidth.

39. Stimulation system (IS) according to any one of claims 35 to 38, for the stimulation of animal or human nerve(s) and/or muscle(s).

FIG.1

FIG.2

FIG.3

FIG.4

FIG.5

FIG.6

FIG.7

FIG.8

FIG.9

FIG.10A

CTC

S

E — M1

M

FIG.10B

CTC

S

Vref — M2

E — M1

M

FIG.11A

E

M1

S

CCT

M

FIG.11B

E

Vref — M2

M1 — S

M

CCT

FIG.11C

E

M2 — S'

M1 — S

M

CCT

FIG.12A

CTCC

E

C — M1

S

C* — M0

M2

M

FIG.12B

CTCC

Vref   S

C — M1

M0   M3

C* —

E —   M2

M

FIG.12C

Vref   S

C* —   M1   — Vdd

M0   M3

E —   M2

M

CTCC

FIG.13

FIG.14

FIG.15A

RCEC

Vdd ← → Vdd
Vref

M11    M21    Mp1
M12    M22    Mp2
M10    M20    Mp0
...

M ← → M

S[1]    S[2]    S[p]

C*[1]    C*[2]    C*[p]
E

**FIG.15B**

MC

S[1]    S[2]    S[p]

E →    CCT    CTCTP    CTCTP    ......    CTCTP

M ←

CA[1:p][1:q] ←

CA[1][1:q]    CA[2][1:q]    CA[p][1:q]

**FIG.16**

MC

S[1]    S[2]    S[p]

CA[1:p][1:q] →    C[1:m]    Sr[1:m]
RCEC
E    Is

Ie
E →    CCT    CTC

M ← → M

**FIG.17**

K [1]　　K [2]　　K [3]　　K [4]　　　　MC

Csg =CA[1:4][1:12]　　Sr[1:12]　Sr[13:24]　Sr[25:36]　Sr[37:48]

Vdd

Vref

CA[1][1]

Sr[1]

M301

CA[1][2]

M101

M201

Sr[2]

M302

M102

M202

CA[4][12]

Sr[48]

M348

M148

M248

E

M100

RCEC

M00

M01

M02

M03

M04

M

CCT

CTC

Vdd

M

**FIG.18**

USR3 (g1,2)

USR5 (g1,1)

USR1 (g1,3)

USR2 (g1,4)

USR7 (g1,5)

Contrôleur

temps

**FIG.19**

USR3 (g1,2)

USR5 (g1,1)

USR1 (g1,3)

USR2 (g1,4)

USR7 (g1,5)

Contrôleur

retards

zoom

Décalages induits

temps

Date d'émission du message de recalage par le contrôleur

Dates de réception d'un message de recalage par les USRi

**FIG.20**

USR3 (g1,2)

USR5 (g1,1)

USR1 (g1,3)

USR2 (g1,4)

USR7 (g1,5)

Contrôleur

zoom

Recalage avant

Recalage arrière

temps

Dates recalées par les USRi

Dates de réception d'un message de recalage par les USRi

Date d'émission du message de recalage par le contrôleur

**FIG.21**

Fenêtre DDP groupe

| $D_{11}$ | $D_{12}$ | $D_{21}$ | $D_{22}$ | $D_{31}$ | $D_{32}$ | $D_{41}$ | $D_{42}$ |

**FIG.22**

Intervalle temporel

USR3 (g1,2)

USR5 (g1,1)

USR1 (g1,3)

USR2 (g1,4)

USR7 (g1,5)

Contrôleur

temps

*Distribution*
*initiale*

*Glissement*
*USR₅ muette*

*Glissement*
*USR₃ muette*

*Glissement*
*USR₁ muette*

*Plus Glissement*
*USR₂ émet*

**FIG.23**

FIG.24

FIG.25

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02089913 A
- US 5954758 A
- WO 9323114 A